# EUROPEAN PATENT SPECIFICATION

(11) **EP 4 157 038 B1**
(45) Date of publication and mention of the grant of the patent: **23.10.2024**
(21) Application number: 21726659.2
(22) Date of filing: 20.05.2021
(51) Int. Cl.: A46B 15/00, A61N 1/05, A61N 1/06, A61N 1/40

(54) **ORAL CLEANING AND TREATMENT DEVICE**
MUNDREINIGUNGS- UND -BEHANDLUNGSVORRICHTUNG
DISPOSITIF DE NETTOYAGE ET DE TRAITEMENT BUCCAL

(30) Priority: 28.05.2020 EP 20176964
(43) Date of publication of application: 05.04.2023
(73) Proprietor: Koninklijke Philips N.V., 5656 AG Eindhoven (NL)
(72) Inventor: UMMADI, Jyothir, Ganeshwar, Reddy, 5656 AE Eindhoven (NL); JOHNSON, Mark, Thomas, 5656 AE Eindhoven (NL); GOTTENBOS, Bart, 5656 AE Eindhoven (NL); GERHARDT, Lutz, Christian, 5656 AE Eindhoven (NL); BRANDAO SILVA, Priscilla, 5656 AE Eindhoven (NL)
(74) Representative: Philips Intellectual Property & Standards
(86) International application number: PCT/EP2021/063406
(87) International publication number: WO 2021/239553

(56) References cited:
- EP-A1- 3 104 807
- EP-A1- 3 104 807
- EP-A1- 3 386 346
- EP-B1- 3 104 807
- EP-B1- 3 386 346
- WO-A1-2007/121760
- US-A1- 2005 064 370
- US-B2- 10 201 701

## Description

### FIELD OF THE INVENTION

The present invention relates to an oral cleaning and treatment device for configuring operation settings of the same, in particular an oral cleaning device employing electromagnetic energy for a cleaning and/or treatment function.

### BACKGROUND OF THE INVENTION

Radio-frequency (RF) electromagnetic emissions can be used to provide a cleaning function in the oral cavity. In particular, an oral cleaning device can include a cleaning unit, such as a head portion, for insertion into an oral cavity of a user, which cleaning unit portion includes one or more electrodes or coils coupled to an RF drive signal generator. The signal generator drives the electrodes or coils with an RF signal generator which causes an RF alternating field to be generated.

When the RF field interacts with surfaces of the teeth and gums, it provides a cleaning function by changing surface properties of surfaces in the mouth. In particular, RF fields generated in this way can remove dental plaque, and also dental calculus. Staining of teeth can also be reduced.

US10201701B2 describes a prior art electric toothbrush. The toothbrush comprises a platen, an RF generator, two RF electrodes, and a dielectric barrier situated between the two RF electrodes in the form of a silicone strip. The toothbrush also includes bristles. The dielectric barrier has a height which extends close to the level of the distal tips of the brush bristles. The barrier forces the RF field created between the electrodes to extend to the top of the barrier, thus reaching the area where the bristles engage with the surfaces of the teeth and gums in use. In this way, the barrier is believed to shape the RF field to bring it closer to the tooth or gum surfaces.

The distal tips of the brush bristles are rubbed against the surfaces of teeth in the usual mechanical manner to clean the teeth, while at the same time, the RF electrodes emit an RF field which provides an enhanced cleaning or tissue treatment function at the same surface which is being brushed, at the level of the tips of the bristles.

Developments in the field of RF oral cleaning devices are generally sought.

### SUMMARY OF THE INVENTION

The invention is defined by the independent claims. The dependent claims define advantageous embodiments.

It has been realized by the inventors that one of the drawbacks of existing RF-based oral cleaning devices is that the RF field generated by the device is homogenous and applies a substantially uniform RF field shape and intensity at every location in the oral cavity. However, for many oral cleaning or treatment applications, it would be useful to be able to vary the field shape so that for example, high intensity locally focused RF fields (e.g. with high local field strength gradients) could be used to treat regions of localized calculus. Such fields could work better at dissolving the minerals, improving the cleaning effect. For example, locally high field gradients may cause locally varying surface properties which may encourage removal of surface impurities. However, focused fields are less efficient for general tooth cleaning purposes, since they can treat only a small highly localized area at any one time, i.e., the field shape has a smaller electromagnetic field footprint.

Embodiments of the present invention are based on providing an RF-energy based oral cleaning or treatment device having an adjustable RF field (e.g. with an adjustable intensity distribution). Depending upon an intended application, the mode of the device can be changed to thereby change an RF field shape or pattern created by the device, and change the spatial distribution of the RF energy delivered by the device. This changes the cleaning characteristics of the device, allowing the device to adapt between for example localized higher intensity energy delivery and more distributed lower intensity energy delivery. This therefore provides greater flexibility in the cleaning functionality of the device.

The drive signals from the RF generator may be alternating (AC) drive signals.

In at least a first mode, the RF field may have a first spatial intensity distribution. In at least a second mode, the RF field may have a second spatial intensity distribution.

The modes are pre-determined or pre-defined or pre-programmed modes, each corresponding to a pre-defined field spatial intensity distribution. The modes are discrete modes for example.

The device may in some embodiments comprise an actuator, the actuator operable to control a position or movement pattern of the protruding members. The actuator may be operatively coupled with the controller,

The controller may in some embodiments be adapted to configure the mode based on controlling the actuator. Additionally or alternatively, the controller may be adapted to configure the mode based on controlling the RF generator (to implement the change in characteristics of the drive signal or the drive scheme).

More specifically, in some embodiments, the controller is adapted to enable selective switching between the modes, wherein the controller is adapted to switch between the modes based on: controlling the actuator to control the change in position or movement pattern of the protruding members; and/or controlling the RF generator to implement the change in characteristics of the drive signal or the drive scheme.

The controller may selectively control switching between the modes based on (e.g. responsive to) a control signal from a user interface, such as a control button. Thus, switching may be triggered by a user-actuated control signal. In other examples, the switching may be triggered automatically, for example based on an internal control signal or a functional signal, such as a signal from a timing or scheduling module, or an output from a sensor.

By way of example, according to one or more embodiments, a high intensity, local cleaning mode could be provided in which the generated RF field comprises a relatively small, high intensity region localized around the region of each protruding member. A further, more wide-area general cleaning mode could additionally be provided which generates an RF field with a lower intensity, with energy spread over a wider spatial area.

Each mode is arranged for generating an RF field with a different time-average spatial intensity distribution, over a defined time window.

Thus at least a first mode may be configured to generate a first time-average spatial intensity distribution, and at least a second mode may be configured to generate a second time-average spatial intensity distribution.

The spatial intensity distribution in one or more of the modes may be time-dependent (vary as a function of time). Thus, in these circumstances, the spatial intensity distribution referred to above is a time-average spatial intensity distribution. The spatial intensity distribution may change periodically, and the time window may be the duration of one period or cycle of this periodic variation.

In a first mode of the device the spatial intensity distribution is more spatially homogeneous over an area containing the protruding members than in a second mode.

Here, the RF energy field is more spatially distributed in the first mode than the second. For example, the time-average spatial intensity distribution may be more spatially homogeneous that in the second mode.

The field being more spatially homogeneous in the first mode may mean that an average gradient of the field, or a maximum gradient of the field, over a defined area or volume, is lower than in the first mode. For example, the average or maximum field gradient over an area spanning the support surface of the support body may be lower. The average or maximum field gradient may be lower over an area with a boundary defined or demarked by positions of the protruding members in some examples, either in their rest positions or their maximum deflection positions when oscillating.

The device may be configured for generating an RF field spatial intensity distribution which includes one or more intensity peaks or field gradients, and wherein in a first mode the peaks or field gradients are configured to move spatially relative to the support surface as a function of time, and in a second mode the peaks or field gradients are configured to exhibit reduced or no movement relative to the support surface.

The variations in the generated RF field intensity distributions for the different modes can be achieved in a range of different ways. These can be categorized into two broad groups: mechanical means for altering the mode (e.g. causing a change in position or movement pattern of the protruding elements), and electrical or signal means (changing properties of the drive signals or the drive scheme of the RF generator).

Changing the drive scheme can include changing the drive signals supplied to individual electrodes, but also changing the pattern of drive signals supplied to the total set of electrodes comprised by the cleaning unit as a whole, e.g. changing the pattern of electrodes which is active at any given time, or varying drive signal amplitudes of different electrodes at different points across the support body.

The electrical means will first be outlined.

The RF generator is operable to independently control drive signals supplied to different subsets of the protruding members, and wherein changing between the modes is based at least in part on selectively activating or deactivating the electrodes of one or more subsets of the protruding members. Thus, in different modes, electrodes of different subsets of the protruding members are controlled to be active or inactive (based on supplying or not supplying drive signals to these electrodes).

In this set of embodiments, an RF field distribution can be changed based on selectively switching on or off the supply of drive signals to different subsets of the protruding members of the cleaning unit. By appropriately controlling the spatial pattern of protruding member electrodes which are active at a given time, the shape (intensity distribution) of the resulting RF field can be controlled.

The controller may be operatively coupled with the RF generator and adapted to control or co-ordinate the selective activation and deactivation, e.g. based on a set of one or more pre-stored activation drive schemes (activation patterns), each configured for generating an RF field with a pre-defined shape or intensity distribution.

As mentioned above, the second broad group of means for implementing the mode change is mechanical means.

For example, in one group of embodiments, changing between modes comprises controlling or actuating a change in a movement pattern of the protruding members. Broadly, if the protruding members are driven to move in a periodic manner (e.g. oscillate), then the (time-average) RF field intensity distribution becomes more spatially distributed (with a smaller average field or field gradient) over the area of the cleaning unit. If they are stationary, or their movement amplitude is less, the RF field intensity distribution is more locally concentrated, e.g. comprising a local region of relative high intensity around the area of each protruding member, but low intensity outside this area. In the first case, the more distributed field is useful for a general cleaning function, where cleaning over a wider area is desired. In the second case, the more locally focused field is useful for higher intensity cleaning of very local areas.

The device may comprise an actuator for controlling a position or movement of the protruding members, for instance in accordance with an adjustable movement pattern. The actuator may be an electrically powered actuator, or a manually driven actuator.

The controller may be operatively coupled to the actuator, and adapted to control adjustment between modes based on controlling the actuator to change a movement pattern applied to the protruding members.

In accordance with one or more embodiments, each of protruding members may be adapted to be flexible, to permit at least a distal end portion of the protruding member to flex or oscillate relative to a proximal end of the member.

The device may further comprise an actuator, for example in the form of an oscillation mechanism, arranged to induce oscillation of at least distal portions of the protruding members.

Switching between the at least two modes may comprise adjusting a frequency of the oscillation of the protruding members. Here, the change between modes is based on altering properties of a driving oscillation applied to the protruding members.

For example, in at least one mode, an actuation might be applied at the support surface to cause periodic or oscillatory displacement of the proximal end of each protruding member. Due to the flexibility of the protruding members, this induces a periodic or oscillatory motion of the protruding members which causes the RF field to be spread over a wider area in that mode. The protruding members may have a lateral flexibility, so that a lateral oscillatory motion of the member is induced for example. In a further mode, the oscillation may be deactivated or set to a lower level, thereby leading to a more locally concentrated field intensity distribution, suitable for example for local high intensity treatment action.

The device may be configurable in a first mode in which the protruding members have zero oscillation, and at least a second mode in which the protruding members have non-zero oscillation. In the zero oscillation mode, the protruding members may be substantially stationary relative to the support surface. This is useful for generating a localized high intensity field.

Where the oscillation mechanism is arranged to oscillate the support surface of the cleaning unit, changing the oscillation of the protruding members may be based on changing a frequency, amplitude or motion pattern of this oscillation of the support surface.

The oscillation of the support surface, may in general be in any direction, e.g. lateral (e.g. transverse a height dimension of the protruding members) or vertical (e.g. parallel the height axis of the protruding member). Lateral movement may include linear lateral movement (side-to-side) or rotational lateral movement of the support surface for instance. Vertical movement may include tapping motion applied to the support body by an actuation mechanism.

According to one or more embodiments, the cleaning unit may further comprise a plurality of cleaning elements or members, such as bristles or cleaning filaments, for cleaning teeth, extending outward from the support surface. The oscillation of the cleaning unit by the mechanical oscillation mechanism may be arranged to cause oscillation of the bristles. In other words, the oscillation that drives the bristle vibration also drives the movement or oscillation of the protruding members.

The bristles and the protruding members are preferably coupled to the same surface and thus the same oscillation action is able to drive oscillations of both.

According to one advantageous set of embodiments, at least portions of the each of the protruding members exhibit a natural frequency of (e.g. lateral) oscillation relative to the proximal ends, and wherein, in a first mode, said frequency of oscillation of the support surface is set at a frequency which is resonant with said natural frequency, and in a second mode said frequency of oscillation of the support surface is set at a frequency which is not resonant with the natural frequency.

Reference to a frequency resonant with the natural frequency means for example a frequency harmonic with said natural frequency, in other words a frequency configured to cause resonant oscillation of the protruding member. In the second mode, the frequency is selected so that it does not cause resonant oscillation of the protruding member.

Hence in the first mode, resonant oscillation of the protruding members is induced which causes the high intensity regions of the field close to the electrodes to be swept over a wider area. In the second mode, the amplitude of oscillation is reduced, thereby confining the high intensity field treatment to a smaller area or volume, which area thereby receives a greater time-average RF energy delivery.

In advantageous examples, in the second mode, the frequency of oscillation of the support surface is set at a frequency which is anti-resonant with the natural frequency of the protruding members. Anti-resonant means for example anti-harmonic. This means for example it is set at a frequency which is between two successive harmonics of the natural frequency.

This means in the second mode, the oscillation is anti-resonant with the natural frequency of the protruding members, meaning that the protruding members are ideally left stationary (substantially zero amplitude and zero frequency of oscillation).

In accordance with one or more embodiments, each of the protruding members may have a proximal end connected to the support surface, and wherein a region of the support body meeting or surrounding the proximal end of each protruding member has a first set of mechanical resonance frequencies (e.g. a range of resonant frequencies or a discrete set of one or more frequencies), and a remainder of the surface exhibits a second set of resonance frequencies.

In a first mode, the support surface may be driven to oscillate at a frequency resonant or harmonic with a frequency of both the first and second frequency sets or ranges. In a second mode, the support surface may be driven to oscillate at a frequency non-resonant with any of the frequencies of the first frequency set or range and resonant or harmonic with a frequency of the second frequency set or range.

Hence this embodiment follows a similar concept to the resonant protruding member embodiment discussed above, except here it is certain portions of the support structure to which the protruding members are coupled which is configured to undergo resonance at a certain one or more frequencies.

Preferably, in the second mode, the support surface may be driven to oscillate at a frequency which is anti-resonant or anti-harmonic with the set or range of resonance frequencies of the region of the support body leading to or surrounding each of the protruding members.

In accordance with one or more embodiments, the device may comprise a secondary actuation or forcing mechanism arranged to apply a second mechanical oscillation to at least a subset of the protruding members, wherein the second mechanical oscillation is at the same frequency as the oscillation applied to the support surface by the primary oscillation mechanism (discussed above), but out of phase with the first oscillation (preferably in anti-phase). Thus the secondary actuation mechanism effectively applies counterforces to the protruding members to counter the force by the primary device oscillation mechanism (e.g. which is for oscillating the bristles), to reduce or eliminate movement of the at least subset of the protruding members.

In accordance with one or more embodiments, the device may further comprise a magnetic or electromagnetic forcing arrangement, operable to exert a magnetic or electromagnetic force on each of the protruding members, and wherein the switching between the modes is based on adjusting a movement pattern of the protruding members based in part on use of the magnetic or electromagnetic forcing arrangement.

This can be used, alone or in combination with other of the features outlined above, to control position or movement (e.g. amplitude and/or frequency of oscillation) of the protruding members in different modes, in order for instance to change the spatial intensity distribution between the different modes.

For example, in accordance with one or more embodiments, the support surface may be oscillated at a first frequency. This may be used in part for oscillating bristles of the cleaning unit for example. The oscillation of the support surface may in general be in any direction, e.g. lateral (e.g. transverse a height dimension of the protruding members) or vertical (e.g. parallel the height axis of the protruding members). Lateral movement may include linear lateral movement (side-to-side), rotational lateral movement, or eccentric arcuate movement of the support surface for instance. Vertical movement may include tapping motion applied to the support body for instance.

In one mode of the device, the magnetic or electromagnetic forcing arrangement may be controlled to impart a pattern of counterforces on the protruding members in synchrony with the oscillation of the support surface, so as to counter or fully eliminate movement of the protruding members due to movement of the support surface.

The magnetic forcing arrangement in this example applies forces in synchrony (or counter-synchrony) with the support surface oscillation so as to counter the movement of each protruding member that would have otherwise occurred due to the support surface oscillation.

In at least a second mode, the magnetic or electromagnetic forcing arrangement may be deactivated. In the second mode, the protruding members are free to oscillate (e.g. laterally, radially or vertically, or any combination thereof) due to the oscillation of the support surface at their proximal ends, and thus the field intensity distribution is more spatially distributed or homogeneous.

Optionally, in addition to varying a spatial intensity distribution of the field, further electrical characteristics of the generated field can also be varied between modes, for example, a frequency composition of the field, and/or a power or intensity level of the field.

By way of example, in accordance with one or more embodiments, the RF generator may be adapted to supply a pulsed RF drive signal to the electrodes, and wherein switching between the at least two modes includes controlling a change in a duty cycle of the pulsed RF drive signal. This can be done for example in combination with mechanical or electrical modification of RF field shape discussed above.

By controlling the duty cycle, it is possible to adjust the overall intensity of cleaning or treatment.

In accordance with one or more embodiments, switching between at least two modes may include adjusting a frequency composition of the drive signals supplied to the electrodes.

In at least one set of embodiments for example, in at least one mode, the RF generator may be configured to generate a drive signal comprising a periodically repeating cycle or sequence of frequencies. This may be a unidirectional scan or cyclic sweep of frequencies for example. Optionally, in a further (first) mode, the drive signal may be a mono-frequency drive signal (one frequency only).

In accordance with one or more embodiments, the controller may be adapted to determine a position and/or orientation of the cleaning unit, and wherein switching between the modes is triggered based on the determined position or orientation information. The controller may determine a position of the device relative to a particular one or more locations in the mouth, or relative to a target treatment area for example.

Additionally or alternatively, the controller may be adapted to trigger switching between modes based on information relating to movement or position of the protruding members. The controller may trigger switching between modes based on a signal from the mechanical oscillation mechanism (arranged to oscillate the support body), the signal indicative of a movement direction of the support surface and/or a movement direction of the protruding members.

The determination may be based on use of a sensor coupled to the controller, for example a position or orientation sensor. The sensor may include an accelerometer and/or a proximity sensor for example. It may include an optical or other electromagnetic sensor. It may include an imaging sensor.

The oral cleaning and/or treatment device may take a number of different forms. By way of non-limiting example, it may be any of: a toothbrush device, a combined brushing and flossing device, an oral irrigator, or a mouthpiece unit with brushing functionality.

The cleaning unit may for example be a head for an oral cleaning device such as, by way of non-limiting example, a toothbrush, a combined brushing and flossing device, an oral irrigator or a mouthpiece unit with brushing functionality. For example, the cleaning device may be a toothbrush device and wherein the cleaning unit is a toothbrush head.

The device may comprise a control unit which comprises the RF generator. It may further comprise a mechanical actuator (e.g. mechanical oscillator) as discussed above. It may further comprise a controller operatively coupled to the RF generator and optionally also a mechanical actuator such as mechanical oscillator. The control unit may be electrically and mechanically coupled to, or releasably coupleable to, the cleaning unit. The control unit may form a body or handle portion of the device.

According to a further aspect, the control unit may be provided by itself, in isolation from the cleaning unit, and adapted to couple in use with the cleaning unit, and operable to control the operation mode of the device when so coupled.

A spatial intensity distribution of the field means a distribution or shape or pattern of field strength, which may incorporate one more field gradients.

A computer program product comprising computer program code is also disclosed but does not form art of the claimed invention, the computer program code being executable on a processor, wherein the code is configured to cause the processor to perform a method in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a front view of a cleaning unit of an example oral cleaning and treatment device;
Fig. 2 shows an example oral cleaning and treatment device;
Fig. 3 shows a plan view of an example cleaning unit with laminar protruding members;
Fig. 4 shows a plan view of a further example cleaning unit;
Fig. 5 shows a further example cleaning unit, comprising bristles;
Fig. 6 illustrates an example cleaning unit in a plurality of different modes, in which different subsets of electrodes are controlled to be active or inactive;
Fig. 7 shows an example cleaning unit with flexible protruding members;
Fig. 8 shows an example oral cleaning and treatment device comprising a mechanical actuation unit;
Fig. 9 shows a further example cleaning unit with an array of flexible protruding members;
Fig. 10 schematically illustrates an example embodiment comprising a cleaning unit which includes material portions with different resonant frequencies;
Figs. 11-14 illustrate a further example cleaning and treatment device comprising a magnetic forcing arrangement;
Fig. 15 shows an example cleaning and treatment device in the form of a toothbrush device; and
Fig. 16 outlines steps of an example operation workflow of an example device according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an oral cleaning and/or treatment device which includes electrodes for generating an RF field for providing an oral cleaning function. The device includes a cleaning unit for receipt in an oral cavity which includes at least a first and second protruding member, which protrude in a same general direction away from a base or support structure. Each protruding member comprises one or more electrodes or antennas for generating an RF alternating electromagnetic field. The device generates an RF field in a space between and around the electrodes by driving the electrodes with an RF signal generator. The device includes means, either mechanical or electrical, for varying a generated RF field (e.g. spatial field intensity or gradient distribution) between different settings. The device is in particular configurable in at least two modes or settings, each associated with a different respective field pattern or spatial intensity distribution. This is valuable for adapting the cleaning or treatment functionality to different required purposes, as discussed below.

In one advantageous group of embodiments, the selectable modes include at least one mode in which an RF spatial intensity distribution includes one or more focused, localized, higher intensity regions, surrounded by lower intensity regions, and a second mode in which an RF spatial intensity distribution, at least measured over a certain window of time (e.g. time-average intensity distribution) is more homogeneous, and the RF energy carried by the field is more widely spread over the area around and between the electrodes.

For example, it is well known that certain specific locations in the oral cavity are more susceptible to formation of plaque and calculus (e.g. teeth in close proximity to salivary glands or lingual front teeth), and consequently to the formation of dental caries. Certain specific locations are more prone to development of gum inflammation such as pericoronitis, for example locations posterior to the last molars, which are difficult to reach with typical mechanical cleaning devices.

Further oral pathologies include chronic dryness of the mouth, which can be caused by decreased secretion from the salivary ducts. Oral pain can be experienced, originating from the trigeminal nerve endings in the mouth. Both of these conditions are thus associated with problems localized to specific areas of the oral cavity.

Although these conditions are currently known, they are difficult to prevent, and once detected, require active oral treatment. In particular, once the root cause of the oral pathology is identified by a dentist, localized treatment of specific spots in the oral cavity is needed to cure the pathological state.

Embodiments of the present invention propose to change the mode of RF treatment to permit application for a variety of local treatment needs.

Thus embodiments of the present invention allow the RF treatment to be optimized to the specific clinical pathology, while avoiding over-exposing other areas of the oral cavity with RF energy.

As will be discussed in more detail below, embodiments can enable, in at least one mode, targeted higher intensity treatment of specific target zones in the oral cavity known to be associated pathologies. These locations may include for instance:
- secondary caries, where biofilms grow at the interface of a dental filling and a tooth;
- pericoronitis: inflammation of the gums posterior to the last molars (both maximal and mandibular);
- lingual mandibular incisors, which typically collect most calculus;
- the trigeminal nerve endings in the oral cavity;
- the salivary ducts.

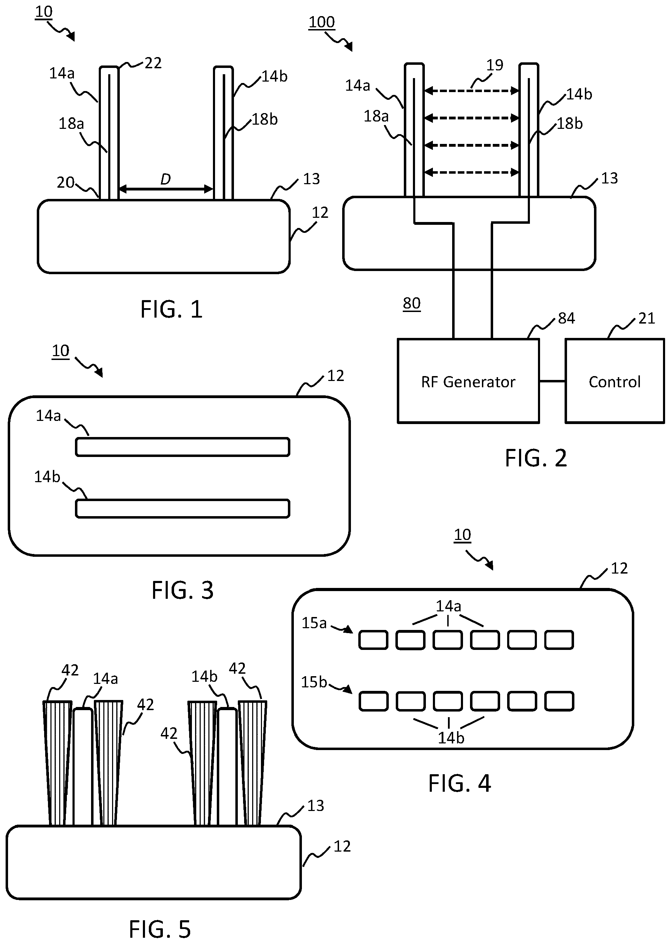

Fig. 1 and Fig. 2 schematically illustrate an example an oral cleaning and/or treatment device according to one or more embodiments. The device 100 comprises at least a cleaning unit 10 and an RF generator 84. Fig. 1 shows the cleaning unit 10. Fig. 2 shows the cleaning and/or treatment device 100 comprising the cleaning unit and RF generator.

The cleaning unit 10 comprises: a support body 12, such as a platen. The support body has an upper face which provides a support surface 13 from which protruding members 14, 14b extend. In particular, the cleaning unit comprises a first protruding member 14a, extending outward from a support surface 13 of the support body, and comprising one or more first electrodes 18a. The cleaning unit further comprises a second protruding member 14b extending outward from the support surface of the support body and comprising one or more second electrodes 18b. The second protruding member is spaced from the first by a spacing, D. Each protruding member 14 has a proximal end 20 which by which it is connected to the support surface 13, and a distal end 22.

The cleaning device further comprises an RF generator 84 arranged to supply an alternating RF drive signal between the first electrodes 18a and second electrodes 18b, to induce an alternating RF electromagnetic field 19 in an area around and between the first 14a and second 14b protruding members. This provides an oral cleaning function when the head is received in an oral cavity. The RF generator applies an alternating potential between the two electrodes. A suitable range of RF frequencies for the alternating electromagnetic field may be any frequency from 3 kHz to 300 GHz for example.

A controller 21 may be operatively coupled to the RF generator 84, and operable to control a drive scheme implemented by the RF generator and/or characteristics of the drive signals generated by the RF generator.

Although Fig. 1 and Fig. 2 show only a single pair of protruding members 14a, 14b and electrodes 18a, 18b, in further examples, more than two protruding members may be provided. There may be provided a plurality of pairs of protruding members, spaced from one another, each member comprising an electrode, and wherein the RF generator is operable to supply a respective alternating drive signal across the electrodes of each pair of protruding members.

The oral cleaning and/or treatment device 100 is adapted to be selectively configurable in at least two different modes. In each mode, the device generates an RF field with a different spatial intensity distribution within an area around and between the protruding members 14a, 14b.

The device is adapted to permit switching between the modes. Implementing switching between the modes may comprise:
- controlling a change in a position or movement pattern of the protruding members, and/or
- controlling a change in characteristics of the drive signal supplied to the electrodes, or changing the drive scheme by which the set of electrodes are driven.

The electrodes 18a, 18b of the protruding members are in this case integrated within the body of each respective protruding member. For example, each protruding member in this case may comprise a laminar (sheet like) protruding member having an electrode integrated therein. In other examples, the electrode may be exposed at a surface of the protruding member or the protruding member itself may be formed from a conductive material to act as an electrode.

The electrode may comprise a conductive element which may take a variety of shapes or forms, for instance planar form, or in the form of a linear wire, or in the form of a wire loop.

Switching between the modes, to thereby change the spatial RF field intensity distribution can be achieved in a variety of different ways, but in general can be categorized into two broad groups: mechanical means for transitioning between modes (e.g. controlling a change in position or movement pattern of the protruding elements), and electrical or signal means (controlling a change in properties of the drive scheme of the RF generator). The various options will be discussed in greater detail to follow.

As mentioned, the device includes a controller 21 for controlling the switching between the modes, for example for controlling signal characteristics of the drive signals generated by the RF generator, or, in some embodiments, by controlling an actuation of the protruding members by an actuator such as a mechanical oscillator or other actuator.

In use, switching between modes can be done in different ways. It could be triggered manually, based on user inputs, by means of a user control element, e.g. a button. In alternative examples, it could be performed automatically, for example based on a position, location or orientation sensing function detecting that the cleaning unit is adjacent a pre-determined region of interest or is held in a pre-determined orientation relative to the region of interest to be treated.

For example, in accordance with one or more embodiments, the device may comprise a position detection means adapted to detect a location of at least a portion of the cleaning unit relative to the region of interest within the oral cavity and/or detect an orientation of the device or cleaning unit. This may be used to detect when the cleaning unit is adjacent to a pre-determined trouble spot in the mouth where higher intensity cleaning would be advantageous. Responsive to detecting the cleaning unit being at such a location, a higher intensity cleaning mode may be automatically activated. When the cleaning unit is moved away from the trouble spot, the lower intensity mode may be automatically activated.

The controller 21 may perform determination of a position and/or orientation of at least the cleaning unit of the device. It may in some examples be operatively coupled with a position or orientation sensor, e.g. an accelerometer and/or an optical sensor for sensing proximity to a surface.

Additionally or alternatively, the controller may be adapted to trigger switching between modes based on information concerning movement or position of the protruding members. The controller 21 may trigger switching between modes based on a signal from a mechanical oscillation mechanism (arranged to oscillate the support body), the signal indicative of a movement direction of the support surface and/or a movement direction of the protruding members.

The cleaning unit 10 is a unit for an RF-energy based oral cleaning or treatment device. In the example of Fig. 1 and 2, the pair of first 18a and second 18b electrodes is suitable for having an alternating potential applied across the pair, for stimulating generation of an alternating RF electromagnetic field between the electrodes.

However, more generally the electrodes may be any form of conductive element suitable for receiving a drive signal and to generate RF energy. For example, one or more electrodes may be provided in the form of a loop for inductively generating an alternating field. Drive signals may be applied across pairs of electrodes, as in the example of Fig. 1, or supplied to individual elements such as coil electrodes in the form of a drive current for instance.

The cleaning unit 10 is for being received within the oral cavity of a user for performing an oral cleaning and/or treatment function. The cleaning unit 10 may be for example a head for an oral cleaning device such as a toothbrush or other device, or may be a different form of cleaning unit, such a brushing arch of a mouthpiece unit for fitting into the mouth.

Optionally, the cleaning unit 10 may further comprise a dielectric barrier member positioned between the first 14a and second 14b protruding members. However this is not essential since in embodiments of the present invention, the field intensity distribution can be actively adjusted so that a physical element for guiding the field pattern (as discussed above) is not essential.

Each of the modes of the device 100 is arranged for generating an RF field with a different time-average spatial intensity distribution, over a defined time window.

Thus a first mode are configured to generate a first time-average spatial intensity distribution, and at least a second mode is configured to generate a second time-average spatial intensity distribution. The spatial intensity distribution in one or more of the modes may be time-dependent (vary as a function of time). Thus, in these circumstances, the spatial intensity distribution referred to above may be a time-average spatial intensity distribution. The spatial intensity distribution may change periodically, and the time window may be the duration of one period or cycle of this periodic variation.

In a first mode of the device 100 the spatial intensity distribution may be more spatially homogeneous over an area containing the protruding members 14a, 14b than in a second mode. Here, the RF energy field is more spatially distributed in the first mode than the second. The area referred to may be the whole area of the support surface 13, or a smaller area, for instance an area extending between the two protruding members 14a, 14b, and bounded by those members.

The spatial homogeneity may be measured by the ratio of the highest value of field intensity or field strength to the lowest field intensity of field strength, e.g. within said area. It may be measured by the (maximum) field gradient, or a further statistical value relating to field strength (e.g. interquartile range of field strength).

For example, the time-average spatial intensity distribution may be more spatially homogeneous in the first mode than in the second mode. This means that over a defined time window, the spatial RF energy field is more evenly spread than in the second mode. In other words, the spatial intensity distribution as a function of time, or over a time window, is more spatially homogeneous than in the second mode.

A peak intensity value within the time-averaged RF field may be lower in the first mode than in the second mode.

For example, a high intensity, local cleaning mode may be provided by the second mode in which the generated RF field comprises a relatively small, high intensity region localized around the region of each protruding member. A further, more wide-area general cleaning mode is provided by the first mode which generates an RF field with a lower intensity, with energy spread over a wider spatial area.

Thus certain embodiments of the invention may permit switching between a more highly focused RF energy field (with larger field gradient) and a less focused, more distributed field (with smaller field gradient) depending upon the desired application.

Regarding the first mode, the more distributed field is useful for a general cleaning or treatment function, where cleaning or treatment over a wider area is desired. In the second case, the more locally focused field is useful for higher intensity cleaning of very local areas, such as a space between a filling and the remaining tooth where bacteria and calculus can accumulate. This represents just one option for the different modes, and various other modes could also be implemented and will be discussed further below.

The device 100 may be configured for generating an RF field spatial intensity distribution which includes one or more intensity peaks or field gradients, and wherein in a first mode the peaks or field gradients are controlled to move spatially relative to the support surface as a function of time, and in a second mode the peaks or field gradients are controlled to exhibit reduced or no movement relative to the support surface.

The peaks in field strength or intensity may correspond to local intensity peak concentrations around the electrodes 18a, 18b for example.

Here, the spatial intensity distribution is time-dependent and varies as a function of time. A more spatially-distributed, lower average intensity field (with smaller average field gradients) can be generated by periodically moving a field pattern containing local spatial peaks over an area between and around the protruding members 14a, 14b. This means that over the given time window, the time average spatial intensity distribution is more spatially homogeneous and the RF energy is more spatially distributed. By contrast, where for instance localized high intensity treatment is desired, in the second mode, the local spatial peaks can be controlled to remain stationary.

The field intensity distribution may undergo periodic changes over a time window.

The switching between the modes in this case may for instance be based on implementing a physical movement pattern of the protruding members 14a, 14b, e.g. in a periodic or oscillatory fashion, or may for instance be based on varying a spatial pattern of an array of protruding members which are active and non-active at a given time. Both examples are discussed in more detail below.

In the above example, the switching between at least two of the modes changes a spatial homogeneity of the field, i.e. a spread of the RF energy over the area or volume of the field. However, additionally or alternatively, modes may be comprised by the device 100 whose intensity distribution differs in terms of an average or overall intensity level of the field, e.g. by increasing output power across the whole field. Thus, the device can be switched from a higher intensity treatment mode (higher power output) to a lower intensity mode (lower power output).

The variations in the generated RF field intensity distributions for the different modes can be achieved in a range of different particular ways. The various options will be discussed in greater detail to follow. Different options for the structure of the cleaning unit 10 will first be briefly discussed.

According to some embodiments, each protruding member 14a, 14b may comprise a laminar (flat, sheet-like) member. This example is illustrated in Fig. 3 which shows a top-down (plan) view of an example cleaning unit 10. Each laminar protruding member in this example defines a plane, and the laminar members are arranged with their respective planes in parallel, and with a major planar surfaces of the members facing one another. In the example of Fig. 3, the protruding members are flat, and extend linearly, but in other examples, they may have a curved laminar shape for example. For example, the protruding members may be provided in the form of concentric rings or arcs in some embodiments.

There are different options for the configuration or arrangement of the protruding members 14a, 14b. The cleaning unit 10 may comprise a single first and second protruding member, which may act as a pair of electrodes (anode and cathode pair). An alternating driving signal may be applied between the pair of electrodes for example.

In further examples, a plurality of first protruding members and a plurality of second protruding members may be provided, each first protruding member comprising at least one respective electrode, and each second protruding member comprising at least one respective electrode. Thus, a plurality of pairs of electrodes may be provided.

An example is shown in Fig. 4 which shows a top-down (plan) view of an example cleaning unit. Here, the cleaning unit 10 comprises a plurality of first protruding members 14a, arranged in a first spatial group 15a, and a plurality of second protruding members 14b, arranged in a second spatial group, the first spatial group arranged spaced from, and facing, the second spatial group. In this example, each spatial group takes the form of a linear spatial group, in particular a line. However, this is not essential, and other configurations are possible, such as each spatial group comprising a cluster having a different shape.

There are different options for the form of the oral cleaning unit 10, depending upon the intended application.

In one set of embodiments, the cleaning unit comprises a plurality of mechanical cleaning elements such as bristles for cleaning teeth. An example is shown in Fig. 5.

The cleaning unit 10 comprises a plurality of bristles 42, for example arranged in bundles or tufts, extending outward from the support surface 13 of the cleaning unit. The cleaning unit may comprise a platen 12, which provides a support body, and wherein a face of the platen forms the support surface.

This set of embodiments may be suitable for example for cases where the cleaning unit 10 is a head for a toothbrush device, for example adapted to couple to a body portion of the toothbrush device, e.g. removable coupling. However, other types of oral cleaning devices may also include bristles 42, such as brushing arches of brushing mouthpiece units which fit into the mouth, or combined brushing and flossing devices with a fluid emitting nozzle in combination with the RF energy emitting elements.

In the example of Fig. 5, each of the protruding members 14a, 14b is surrounded on both lateral sides by bristles 42. The bristles may cover substantially the whole area of the support surface in some examples, possibly excluding an outer peripheral region of the surface. However, this is not essential. Bristles may be provided on just one lateral side of the protruding members, for example with no bristles in the space between the members.

Preferably, the protruding members 14a, 14b may extend to a distal height which is shorter than the distal height of the bristles 42.

As mentioned above, the variations in the generated RF field intensity distributions for the different modes can be achieved in a range of different ways. These can be categorized into two broad groups: mechanical means for altering the mode (e.g. causing a change in position or movement pattern of the protruding elements 14a, 14b), and electrical or signal means (changing properties of the drive scheme of the RF generator 84).

The electrical means will first be outlined.

In accordance with one or more embodiments, the RF generator may be operable to independently control drive signals supplied to different subsets of the protruding members, and wherein changing between the modes is based at least in part on selectively activating or deactivating drive signals to electrodes of one or more subsets of the protruding members.

In this set of embodiments, an RF field distribution can be changed based on selectively switching on or off the supply of drive signals to different subsets of the protruding members of the cleaning unit. By appropriately controlling the spatial pattern of protruding member electrodes which are active at a given time, the shape (intensity distribution) of the resulting RF field can be controlled.

As discussed, the controller may be operatively coupled with the RF generator. This may be adapted to control or co-ordinate the selective activation and deactivation, e.g. based on a set of one or more pre-stored activation drive schemes (activation patterns), each configured for generating an RF field with a pre-defined shape or intensity distribution.

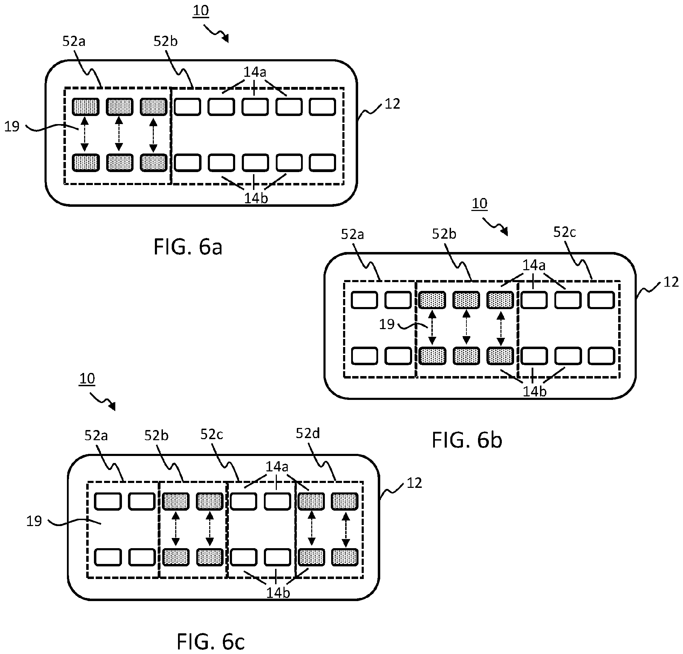

Fig. 6 schematically illustrates this embodiment. Fig. 6a shows the cleaning unit 10 with the device in a first mode. Fig. 6b shows in the cleaning unit with the device in a second mode. Fig. 6c shows the cleaning unit with the device in a third mode.

In each mode, the controller is adapted to apply a different activation drive scheme or addressing scheme to the plurality of protruding members, for controlling a different selection of protruding member 14a, 14b electrodes to be active or inactive. Active members are shown as shaded, while inactive members are shown as non-shaded.

In the first mode of Fig. 6a, a first subset 52a of protruding members 14a, 14b is supplied with drive signals for driving their respective electrodes to generate an RF field 19. At the same time, a second subset 52b of protruding members is not supplied with drive signals, such that their respective electrodes are inactive, and no field is generated between these members. This results in generation of an RF field in the first mode which has a peak intensity in a spatial region covered by the first subset 52a of protruding members. In this example, each subset includes a plurality of pairs of first 14a and second 14b protruding members comprising electrodes which are either actively driven, or inactive.

In the second mode of Fig. 6b, electrodes of a first subset 52a of protruding members 14 and a third subset 52c of protruding members is inactive, while a second subset 52b, between the first and second, is supplied with drive signals by the RF generator, and is active. Thus an RF field 19 is generated which is has a peak intensity in a middle region covered by the second subset 52b of electrodes. In this example, each subset includes a plurality of pairs of first and second electrodes which are either actively driven, or inactive.

In the third mode of Fig. 6c, electrodes of a first 52a and third 52c subset of protruding members are supplied with drive signals from the RF generator, while those of a second 52b and fourth 52d subset are not. The plurality of protruding members 14 thus includes alternating subsets of active and inactive members. Thus a resulting RF field 19 is generated which has alternating regions of high and low field intensity, with peaks in the regions covered by the second 52b and fourth 52d subsets of protruding members. In this example, each subset includes a plurality of pairs of first and second electrodes (comprised by the first 14a and second 14b protruding members) which are either actively driven, or inactive.

The different activation drive schemes shown in Fig. 6 represent illustrative examples only, and in further examples, different modes may be characterized by any desired pattern of active and inactive protruding member electrodes.

In accordance with one or more embodiments, the changing between the modes may comprise adjusting a drive scheme or duty cycle of drive signals supplied to different subsets of protruding member electrodes. For example, electrodes of different subsets of protruding members may be supplied with drive signals having different frequencies or different amplitudes, or different pulse duty cycles, to thereby configure a field shape or pattern or intensity distribution of the resulting field.

Optionally, other electrical characteristics of the generated field can also be varied between modes, for example, a frequency composition of the field, and/or a power or intensity level of the overall field.

By way of example, in accordance with one or more embodiments, the RF generator 84 may be adapted to supply a pulsed RF drive signal, and wherein switching between the at least two modes includes controlling a change in a duty cycle (frequency) of the pulsed RF drive signal. This can be done for example in combination with mechanical and/or electrical modification of RF field shape.

By controlling the duty cycle, it is possible to adjust the overall intensity of cleaning between lower intensity, e.g. for normal cleaning, and high intensity for treating local trouble spots. High intensity pulses can thus provide high intensity cleaning in a target region or location.

An RF pulse scheme with adaptable duty cycle is useful for a number of clinical applications. For example, RF pulse treatment can be used to stimulate nerves in the oral cavity, to thereby relieve nerve pain. Pulse treatment can also be used stimulate the salivary ducts, to thereby relieve chronic mouth dryness.

Adjustment of the duty cycle allows the intensity of treatment to be adjusted. This can avoid over simulation of oral tissue. Alternating between higher and lower duty cycle frequencies can also prevent overheating of the device 100.

Change of a pulse duty cycle can be used alone or in combination with other possible implementation options (discussed below) when switching between modes.

Implementation and adjustment of the duty cycle may be controlled by the RF generator 84 itself, or the controller may be operatively coupled with the RF generator and adapted to control the duty cycle between different selectable modes.

By way of example, the total output power of the generated field may according to advantageous embodiments, be within a range of 0.1W to 15W, for example 0.1W to 10W. These ranges are safe for use in a mouth of a user and is sufficient for providing treatment effects discussed above.

In accordance with one or more embodiments, switching between the at least two modes may comprise adjusting a frequency composition of the drive signals supplied to the electrodes.

In at least one set of embodiments for example, in at least one mode, the RF generator 84 may be configured to generate a drive signal comprising a periodically repeating cycle or sequence of RF frequencies. This may be a sweep of frequencies for example.

Preferably in at least one further mode, the drive signal may be a mono-frequency drive signal (one frequency only).

Additionally or alternatively, switching between the at least two modes may comprise adjusting the frequencies included in the temporal cycle or sweep.

The cycle or sequence of frequencies may comprise a sweep of frequencies (e.g. continuous sweep of frequencies). Alternatively, it may comprise a sequence of discrete frequencies stepped through in series.

Use of a frequency sweep or sequence in this way is useful for a number of clinical applications. For example, it can be useful for stimulating nerves in the oral cavity, to thereby relieve nerve pain. It can also be useful for stimulating the salivary ducts, to thereby relieve chronic mouth dryness.

As mentioned above, the second broad group of means for implementing the mode change is mechanical means.

In one group of embodiments in particular, changing between modes is based on altering a movement pattern of the protruding members 14a, 14b.

An actuator may be provided adapted to control a position or movement pattern of the protruding members. This may be operatively coupled to the controller for example, the controller adapted to control mode changes based on controlling the actuator.

In accordance with at least one set of embodiments, to implement the movement, each of the plurality of protruding members may be adapted to be flexible, to permit at least a distal end portion of the protruding member to flex or oscillate relative to a proximal end 20 of the member. This may allow for example sweeping lateral movement of the distal ends 22 of the protruding members, where the flexibility is in a lateral direction (meaning a direction perpendicular a height axis of the protruding member).

The member itself may be formed of a flexible material to facilitate this flexibility (e.g. passively by means of elastic body deformation or actively by actuation such as local stiffening or hardening of the distal end 22), or the member may be connected to the support surface via a connecting element which permits resilient displacement of the protruding member relative to the support surface, e.g. laterally or axially. This may be a flexible or resilient connecting member, or it may be a swivel joint or other connection element which is adapted to exert a bias on the proximal end of the member to bias it toward a neutral position.

The device 100 may further comprise an actuator for applying a movement to the protruding members, for example a periodic movement such as an oscillation. The actuator may be an oscillation mechanism 82 arranged to induce oscillation of at least distal portions of the protruding members.

Switching between the at least two modes may be based on adjusting a frequency of the oscillation of the protruding members.

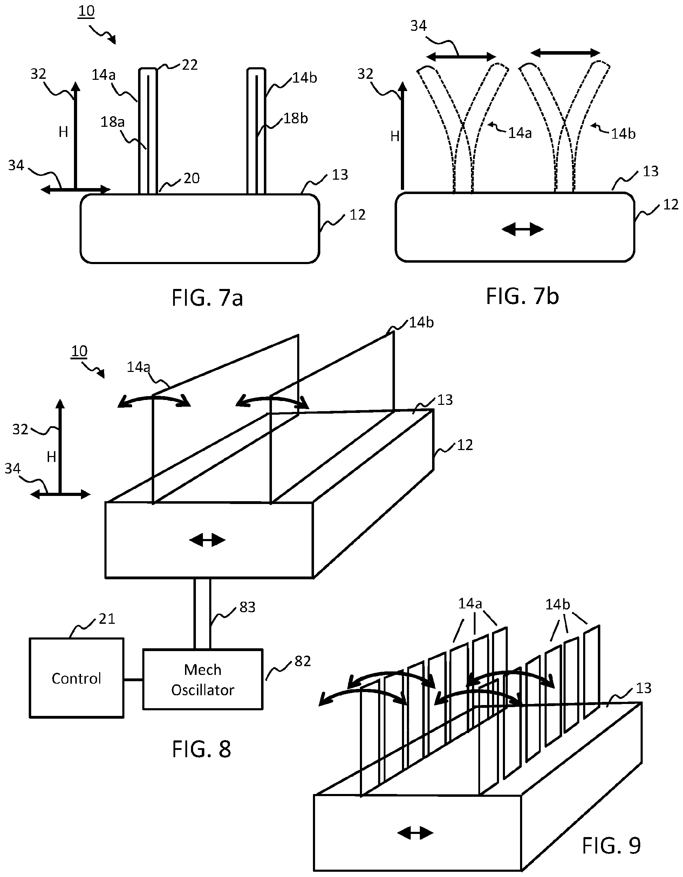

An example is shown in Fig. 7 and Fig. 8. Fig. 7 shows the oral cleaning unit 10, and Fig. 8 shows components of the oral cleaning or treatment device, including an actuator in the form of a mechanical oscillation mechanism 82 (e.g. vibrator, drive train). The oscillation mechanism in the example of Fig. 8 is be adapted to apply a lateral oscillation to the support body 12. However, in other examples, the oscillation mechanism may be adapted to apply a vertical oscillation (e.g. tapping motion), or a rotational (radial) oscillation in some examples.

In Fig. 7 and Fig. 8, each of the protruding members is adapted to be flexible in a lateral direction 34, the lateral dimension being perpendicular a height dimension 32 (H) of the member, to permit at least a distal end portion of the protruding member to flex or oscillate laterally relative to a proximal end 20 of the member. The height axis of each protruding member extends from a proximal end 20 to a distal end 22.

The change between modes may be based on altering properties of a driving oscillation applied at proximal ends 20 of the protruding members 14a, 14b. The oscillation may be applied to the support body 12 itself so as to cause oscillation of the support surface 13 to which the protruding members are connected.

For example, in at least one mode, a suitable actuation or vibration might be applied at the support surface 13 to cause oscillatory displacement of the proximal end 20 of each protruding member. Due to the lateral flexibility of the protruding members, this induces a lateral oscillatory motion of distal portions of the protruding members 14 which causes the RF field to be spread over a wider area in that mode, and causes oscillatory variation in the position of the high intensity region(s) of the RF field. This can therefore be used to implement a wider area, distributed cleaning mode. In a further mode, the oscillation may be deactivated or set to a lower level, thereby leading to a more locally concentrated field intensity distribution, suitable for example for local high intensity cleaning action.

The frequency can be zero in some modes, so that the protruding members 14 are not oscillated, but kept stationary.

The distal end 22 of each protruding member may be adapted to flex or oscillate laterally relative to the proximal end 20 responsive to lateral oscillation of the proximal end.

By way of example, each protruding member may be adapted to be flexible with a maximum angle of lateral deflection of at least ± 5 °. Each protruding member may, by way of non-limiting example, have a maximum angle of lateral deflection for example anywhere from ± 5 ° to ± 45 °.

As discussed above, each protruding member 14, 14b may be in the form of a laminar member. An example is illustrated in Fig. 8 which shows the cleaning unit 10 in perspective view.

As shown, the laminar protruding members 14a, 14b, each extend from a line on the support surface 13, and thus define an elongate, linear footprint on the support surface. In this is example, the two protruding members are arranged facing one another.

A plane of each laminar protruding member 14a, 14b in this example extends perpendicular the lateral dimension 34, so that each protruding member is adapted to be flexible in a direction perpendicular to the plane which it defined. The direction of deflection of the protruding members is illustrated by arcuate arrows in Fig. 8.

A laminar shape of the protruding member 14 enhances flexibility, since a flexural rigidity of the member is reduced in a direction perpendicular the laminar plane. This also helps to constrain or define the direction of lateral flexibility of the member, i.e. the dimension perpendicular the plane of the laminar member.

A further example cleaning unit 10 is shown in Fig. 9.

Here, the cleaning unit 10 comprises a plurality of first protruding members 14a, arranged in a first row, and a plurality of second protruding members 14b, arranged in a second row, the first row arranged spaced from and facing the second row.

Breaking the protruding members 14 up into multiple smaller protruding members can help to tune the desired oscillation characteristics for the members. Smaller members may exhibit for example a smaller amplitude of deflection due to their smaller mass. They may have a different natural frequency of lateral oscillation, which may be useful for tuning a resonant frequency of oscillation of the electrodes. This will be discussed in greater detail later.

To enable the flexibility, each of the protruding members 14a, 14b may comprise a flexible material. In this example, each member is at least partially formed by a flexible material. Each member may for example consist entirely of a flexible material or only a portion may be formed of a flexible material.

By way of one possible example, each protruding member may comprise a nylon material, or may comprise an elastomeric material such as rubber.

In other examples, the flexibility may be achieved in a different way, for example with a flexible pivot or junction member connected between the proximal end of the protruding member and the support surface.

According to one set of embodiments, each of the protruding members may comprise one or more conductive electrode elements, at least partially coated with a flexible polymer. This is advantageous since the conductive electrode core is insulated around its side faces by the polymer, enhancing safety and/or reducing the risk of short circuits with the other protruding member.

In alternative examples however, the protruding member 14 may comprise a flexible polymer core, coated with a conductive outer layer to provide the electrode. Other examples include laminar protruding members formed of laminates of metal and polymer.

In further examples, the protruding member 14 may be formed of a conductive polymer material. Such materials typically have greater elastic properties compared to metal, thus allowing them to provide both the conductive properties, for the RF energy transmission, and also the required elastic properties. This would simplify manufacturing since each member could be formed form a single material only, instead of two materials.

However, a further alternative also includes the protruding member 14 being formed of a laminar metal element, the flat, laminar shape providing the necessary lateral flexibility, and the metal material providing the required conductive properties. Any suitable conductive metal could be used. A preferred example may be stainless steel.

The device 100 may be configurable in a first mode in which the protruding members 14a, 14b have zero oscillation, and at least a second mode in which the protruding members have non-zero oscillation. In the zero oscillation mode, the protruding members 14a, 14b are substantially stationary relative to the support surface 13. This is useful for generating a localized high intensity field.

As mentioned above, the actuation or oscillation mechanism 82 may be arranged to oscillate the support surface 13 of the cleaning unit 10 at an adjustable frequency to thereby induce the oscillation of the protruding members 14a, 14b, and wherein switching between the modes comprise adjusting a frequency of oscillation of the support surface.

This is illustrated in Fig. 8 which shows the actuation or oscillation mechanism 82 arranged to apply an oscillation to the support body 12 (e.g. platen) which thereby causes oscillation of the support surface 13.

The actuation or oscillation mechanism 82 may include a motor or actuator for generating an oscillatory motion, and a mechanical coupling element 83, e.g. a drive shaft or other mechanical connector, arranged to mechanically couple the oscillatory motion to the cleaning unit 10. These components may form a drive train for the device.

The controller 21 may be operatively coupled with the mechanical oscillator, and adapted to control a frequency of oscillation of the cleaning unit by the motor of the oscillation mechanism 82. The controller may control switching between the different modes based on controlling the mechanical oscillator.

The oral cleaning unit 10 may comprise a mechanical coupling portion (not shown), e.g. a coupling slot or groove, shaped to receive the coupling element 83 (e.g. drive shaft) of the actuation or oscillation mechanism 82, and arranged to couple the actuation or oscillations of the actuation or oscillation mechanism to the support surface 13.

The mechanical actuation or oscillation mechanism 82 may in particular be arranged to induce a lateral oscillation of the cleaning unit support surface 13. For example, the oscillation may be configured to cause at least distal tips of the protruding members 14 to oscillate laterally. Tip means the terminal point of the protruding member.

It is noted that the use of a mechanical actuation or oscillation mechanism 82 is not the only means for inducing movement of the protruding members. For example, alternatively, a dedicated actuator or driver could be provided arranged to directly exert biasing or urging forces on the protruding members to induce movement. This could induce oscillatory movement, or a different pattern of movement. It may simply change a position of the protruding elements to thereby reconfigure the RF field pattern which is generated.

The cleaning unit 10 may further comprise a plurality of cleaning elements 42, such as bristles for cleaning teeth, extending outward from a surface of the cleaning unit. The oscillation of the cleaning unit by the mechanical actuation or oscillation mechanism 82 may be arranged to cause oscillation of the bristles 42. In other words, the oscillation that drives the bristle vibration also drives the movement or oscillation of the protruding members 14a, 14b.

The bristles 42 and the protruding members 14a, 14b are preferably coupled to the same surface 13 and thus the same oscillation action is able to drive oscillations of both.

According to one advantageous set of embodiments, at least portions of each of the protruding members may exhibit a natural frequency of oscillation (e.g. lateral oscillation) relative to proximal ends 20 of the members, and wherein, in a first mode, said frequency of oscillation of the support surface 13 is set at a frequency which is resonant with said natural frequency, and in a second mode said frequency of oscillation of the support surface is set at a frequency which is not resonant with the natural frequency.

Reference to a frequency resonant with the natural frequency means for example a frequency which matches or is harmonic with said natural frequency, in other words a frequency configured to cause or induce resonant oscillation of the protruding member 14, i.e. so that the protruding member is at resonance. In the second mode, the frequency is selected so that it does not cause resonant oscillation of the protruding member.

Hence in the first mode, resonant oscillation of the protruding members 14a, 14b is induced which causes the high intensity regions of the field close to the electrodes 18a, 18b to be swept over a wider area. The amplitude of oscillation of (e.g. the distal tips of) the protruding members (indicated by the arcuate arrows in Fig. 8) is thus maximized in the first mode and furthermore can be larger than the amplitude of the oscillation applied to the support surface 13 of the cleaning unit. In the second mode, the amplitude of oscillation is reduced, thereby confining the high intensity field treatment to a smaller area, which area thereby receives a greater time-average intensity of radiation.

The second mode is thus useful for localized, targeted, high intensity RF treatment, while the first mode is useful for more general or wider-area treatment.

As mentioned, the cleaning unit 10 may further comprise a plurality of bristles 42 for cleaning teeth, extending outward from a surface 13 of the cleaning unit 10, and the oscillation of the cleaning unit by the mechanical oscillation mechanism may be arranged to cause oscillation of the bristles.

Thus, the above embodiment allows the mode of RF treatment which is delivered to be fully decoupled or separated from the oscillation action of the bristles 42. In particular, the shape and intensity distribution of the RF energy field delivered by the device 100 can be controlled independently of the movement action of the bristles, through the variation of the oscillation frequency of the support body 12, and coordinating this according to a mechanical resonance frequency of the protruding members 14.

The resonance frequency (natural frequency) of each protruding member 14a, 14b depends upon the material properties of the protruding member, as well its dimensions (height, width, thickness). The natural frequency of lateral oscillation of each member 14 can than thus be tuned based on adjusting these parameters in manufacture.

To increase a natural amplitude of oscillation, the distal tips 22 of the protruding members 14a, 14b may be designed to have a greater mass density than a remaining portion of the member, so that the member is weighted toward its tip (the center of mass is closer to the distal end 22 of the member than to the proximal end 20).

The natural oscillation frequency of the protruding members 14 is also determined by their width. Thus a higher natural frequency might be achieved by providing a plurality of shorter-width protruding members, for example as shown in the example of Fig. 9 described above.

By way of non-limiting example, the oscillation applied to the support body by the oscillation mechanism 82 may be anywhere in the range 50-500 Hz. The natural resonance frequency of the protruding members may be adapted to be a sub-range of the range of oscillation frequencies.

In advantageous examples, in the second mode, the frequency of oscillation of the support surface 13 may be set at a frequency which is anti-resonant with the natural frequency of the protruding members 14a, 14b.

Anti-resonant means for example anti-harmonic. This means for example it is set at a frequency which is between harmonics of the natural frequencies, e.g. between the first and second harmonics.

This means in the second mode, the oscillation is anti-resonant with the natural frequency of the protruding members 14a, 14b, meaning that the amplitude of movement of the protruding members is reduced and the members are ideally left stationary.

In accordance with a further set of embodiments, each of the protruding members 14a, 14b may have a proximal end 20 connected to the support surface 13, and wherein a region 42 of the support body 12 meeting or surrounding the proximal end of each protruding member has a first set of mechanical resonance frequencies, and a remainder 44 of the surface has a second set of resonance frequencies.

In a first mode, the support surface 13 may be driven to oscillate at a frequency in which both regions are resonant. In a second mode, the support surface may be driven to oscillate at a frequency non-resonant with the first region and resonant with the second region. Hence this embodiment follows a similar concept to the resonant protruding member embodiment discussed above, except here it is the support structure 12 to which the protruding members 14a, 14b are coupled which is configured with the possibility to undergo resonance at different frequencies. In particular, the support structure 12 is provided with different material regions with different resonant frequencies or spectrums.

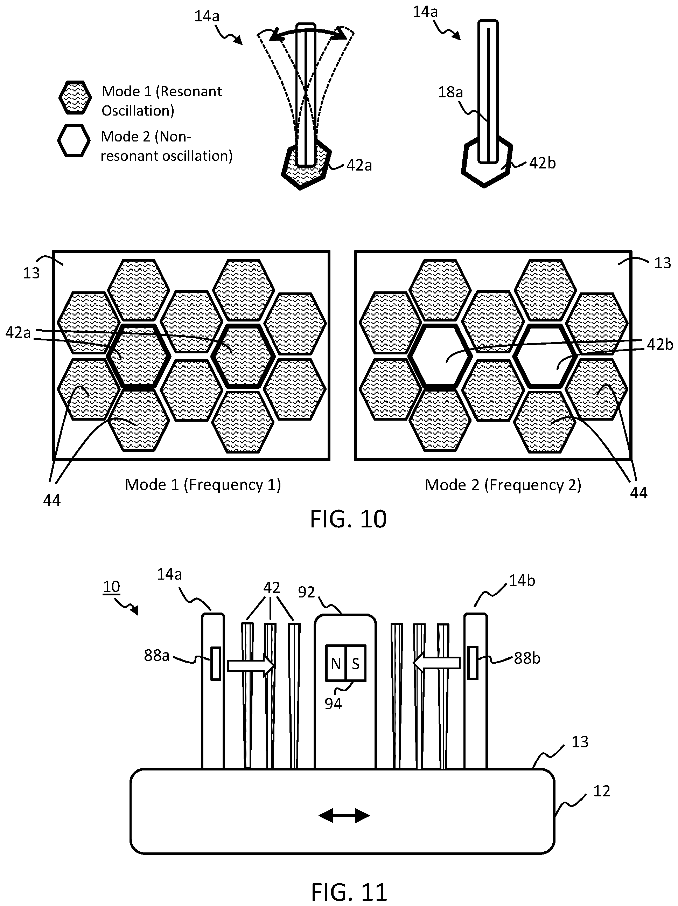

The concept is illustrated in Fig. 10 which shows one of the protruding members 14a and a region 42 of the support surface 13 to which it is connected. This region can be referred to as a unit cell of the support surface. A portion of the support surface, when oscillated at each of a first and second frequency (frequency 1 and frequency 2), is schematically depicted in Fig. 10. Surface regions 42 represent regions or unit cells of the support surface 13 to which proximal ends of the first 14a and second 14b protruding members would be connected. Surface regions 44 shows regions or unit cells of a remaining portion of the support surface. In a first mode ("Mode 1"), the support surface 13 is oscillated at Frequency 1. In a second mode ("Mode 2"), the support surface 13 is oscillated at Frequency 2.

The material regions 42 adjacent to or surrounding each of the protruding members 14a, 14b are configured to exhibit resonant oscillation within a first set of frequencies, and the remaining regions 44 (e.g. from which bristles protrude) are configured to exhibit resonant oscillation at a second, overlapping set of frequencies.

If a distributed, wide-area RF cleaning mode is desired, the support structure 12 can be driven in Mode 1, with an oscillation having a frequency (Frequency 1) which is within the set of resonant frequencies of both sets of support structure regions (within the range of overlap between the resonant frequencies of the two sets of regions). As a result, all of the unit cells of the support surface 42, 44 are induced to oscillate at resonance (indicated by waved lines in each cell in Fig. 10). As a result, the oscillation is induced at proximal ends of the protruding members 14a, 14b, due to the oscillation of regions 42. Therefore, the protruding members are induced to oscillate, leading to a spatially distributed RF intensity distribution.

By contrast, if a local, high intensity cleaning mode is desired ("Mode 2"), the support structure 12 can be driven with an oscillation frequency which is outside the set of resonant frequencies of the regions 42 adjacent the protruding members 14a, 14b, but it is a resonant frequency of the remaining regions 44 of the support structure 12. In this mode, the protruding members are not induced to oscillate with a large amplitude because the units 42 of the support surface 13 to which they are coupled are not oscillating at resonance (as schematically indicated by the clear fill of cells 42b), whereas the bristles can still oscillate at a high amplitude since the regions 44 of the surface to which they are coupled are oscillating at resonance.

Preferably, in the second mode, the support surface may be driven to oscillate at a frequency which is anti-resonant or anti-harmonic with the range of resonance frequencies of the regions 42 of the support body leading to or surrounding each of the protruding members 14a, 14b.

The protruding members 14a, 14b may each have natural or resonant frequencies of oscillation which is within said first set of mechanical resonance frequencies of the regions 42 of the support surface 13 to which the protruding members 14a, 14b are connected. It may have the same set of mechanical resonance frequencies in some examples.

The different resonant frequency ranges of the different regions 42, 44 of the support surface may be achieved according to one or more embodiments through use of acoustic metamaterials.

Metamaterials are an emerging technology and relate to engineered materials characterized by their structure (geometric arrangement of one or more materials) rather than by their material composition. Metamaterials enable achievement of new physical properties and features that are not available in naturally occurring materials, such as a negative refractive index for example.

Acoustic metamaterials can be formed which show negative or near-zero dynamic density and/or elastic modulus. Acoustic metamaterials may be constituted of sub-wavelength unit cells, for example in a periodic arrangement. Acoustic metamaterials are characterized by exhibiting mechanical (acoustic) frequency bands in which wave propagation through the material cannot occur. These nonpropagating frequency bands are known as band gaps of the material. Different units of the material can have different band gaps. These enable non-conventional ways of manipulating energy.

For embodiments of the present invention, acoustic metamaterials enable provision of the support body 12 (e.g. platen) having the different material regions 42, 44 (unit cells) discussed above. A metamaterial support body 12 may be provided in which the unit cells 42 to which the protruding members 14 are connected have a first band gap (a frequency range in which acoustic vibration cannot occur), while the remaining unit cells 44 do not have this band gap, or have a different band gap. In a first mode, the support body can be oscillated at a first frequency which is outside of the band gap of all material regions 42, 44. This results in oscillation of all areas of the support body, including the regions at the base of the protruding members. Thus the protruding members oscillate and a spatially distributed RF field is generated. In a second mode, the support body may be oscillated at a second frequency, which is within the (first) band gap of the regions 42 to which the protruding members are connected, but not within any band gap of the remaining regions 44. This results in oscillation of all regions of the support surface 13 except those at the base of the protruding members. As a result, the protruding members do not oscillate.

In some embodiments, an acoustic metamaterial support body 12 may be provided which comprises one or more material zones which exhibit a band-gap, and wherein a subset of the plurality of protruding members 14 are connected to the support surface 13 within these regions, and further subset are connected to the support surface 13 outside of these regions. This results in a cleaning unit in which spatial zones are defined in which vibration at certain frequencies cannot occur, and thus the subset of protruding members positioned in these zones will not oscillate when the support body is oscillated at a frequency within the band gap. Thus, when the support body is oscillated at these frequencies, a zone of protruding members remains substantially stationary, resulting in a locally higher intensity RF field in these zones.

These metamaterial zones having the bandgap can be positioned at locations on the support surface 13 of the cleaning unit, designed to coincide with certain anatomical feature of structures in the mouth when the cleaning unit is received in the mouth. For example, the device may be a mouthpiece device, and the band-gap zones may be formed at certain regions of the surface of the mouthpiece.

In accordance with an advantageous set of embodiments, the device may include means permitting selective adjustment of the oscillation characteristics of different subsets of the protruding members, to further attune the spatial intensity distribution of the generated RF field. This can be achieved in different ways.

In one set of embodiments, an active mechanical damping element may be provided at a proximal end of each protruding member, adapted to apply an adjustable mechanical damping to the proximal end of each member. In this way, an amplitude of oscillation of different subsets of protruding members, responsive to the oscillation applied by the mechanical actuation or oscillation mechanism 82, may be selectively adjusted. For example, a high level of damping may be applied to a first subset of protruding members, and a low-level or zero damping applied to a second subset, so that the first subset exhibits a reduced amplitude of oscillation relative to the support surface compared to the second subset. The particular subsets of members which are dampened, and the subsets which are not dampened, may be adjusted between different modes (for example by the controller 21), to adjust therefore the spatial intensity distribution of the resulting RF field between different modes.

The damping elements may comprise for example ring elements extending around the base of each protruding member and operable to exert an adjustable radial inward force on the protruding member to exert an adjustable mechanical resistance.

Additionally or alternatively, according to one or more embodiments, the oscillation characteristics of different subsets of the protruding members may be adjusted in different modes based on providing protruding members, different subsets of which exhibit different natural frequencies of oscillation. In this way, different subsets can be induced to oscillate at larger or smaller amplitudes in different modes based on selectively adjusting a frequency of mechanical oscillation applied to the members (e.g. to the support surface) in different modes.

Additionally, or alternatively, according to a further set of embodiments, a secondary actuation or forcing arrangement may be provided arranged to provide a secondary actuation or forcing to at least one or more subsets of the protruding members. This may for example be used to selectively counter a primary oscillation applied to the support surface for oscillating the bristles.

In accordance with one or more embodiments, the oral cleaning or treatment device 100 may further comprise a magnetic or electromagnetic forcing arrangement, operable to exert a magnetic or an electromagnetic force on each of the protruding members, and wherein the switching between the modes comprises adjusting a movement pattern of the protruding members based in part on use of the magnetic or electromagnetic forcing arrangement. The forcing arrangement can be active or passive.

For brevity, the arrangement will be referred to herein as simply a magnetic forcing arrangement.

An example is schematically illustrated in Fig. 11. This shows an example cleaning unit 10 comprising the magnetic forcing arrangement and a pair of protruding members 14a, 14b. The cleaning unit further comprises a plurality of bristle bundles 42 which are schematically depicted in Fig. 11. In this example the forcing arrangement is an active forcing arrangement.

In this example, the magnetic forcing arrangement comprises a further (central) protruding member 92 positioned between the first and second protruding members. This may be dielectric barrier member in some examples, for further shaping the RF field.

One of the first protruding member 14a and further protruding member 92 include a permanent magnet 94, and the other includes a solenoid 88a, positioned in the magnetic field of the permanent magnet. The solenoid may comprise a wire coil or loop for example.

One of the second protruding member 14b and further protruding member also include a permanent magnet 94, and the other includes a solenoid 88b, positioned in the magnetic field of the permanent magnet. The solenoid may comprise a wire coil or loop for example. In this example, a single permanent magnetic is provided in the central protruding member 92 for magnetically coupling a first solenoid 88a in the first protruding member 14a and a second solenoid 88b in the second protruding member 14b.

A resultant magnetic force exerted on each protruding member 14a, 14b is controllable based on controlling a current supplied to the respective solenoid 88a, 88b. The controller may be further arranged to control provision of current to the solenoids.

This can be used, alone or in combination with other of the features outlined above, to control position or movement of the protruding members 14a, 14b in different modes, in order to change the spatial RF field intensity distribution between the different modes.

For example, in accordance with one or more embodiments, the support surface 13 may be oscillated at a first frequency. This may be used in part for oscillating bristles 42 of the cleaning unit for example. In one mode of the device, the magnetic forcing arrangement may be controlled to impart a pattern of (magnetic or electromagnetic) counterforces on the protruding members 14a, 14b in synchrony with the oscillation of the support surface, so as to counter movement of the protruding members 14a, 14b due to movement of the support surface 13.

The oscillation of the support surface 13 may be for the purpose of oscillating bristles 42 comprised by the cleaning unit 10 and extending out of the support surface 13. Due to this oscillation, in the absence of counterforce, the protruding members would also be induced to oscillate. For a locally more focused intensity mode, it is desirable that the protruding members are substantially stationary, or that their oscillation is minimized. The magnetic forcing arrangement applies resultant electromagnetic forces in synchrony (or counter-synchrony) with the support surface oscillation so as to counter the movement of each protruding member that would have otherwise occurred due to the support surface oscillation.

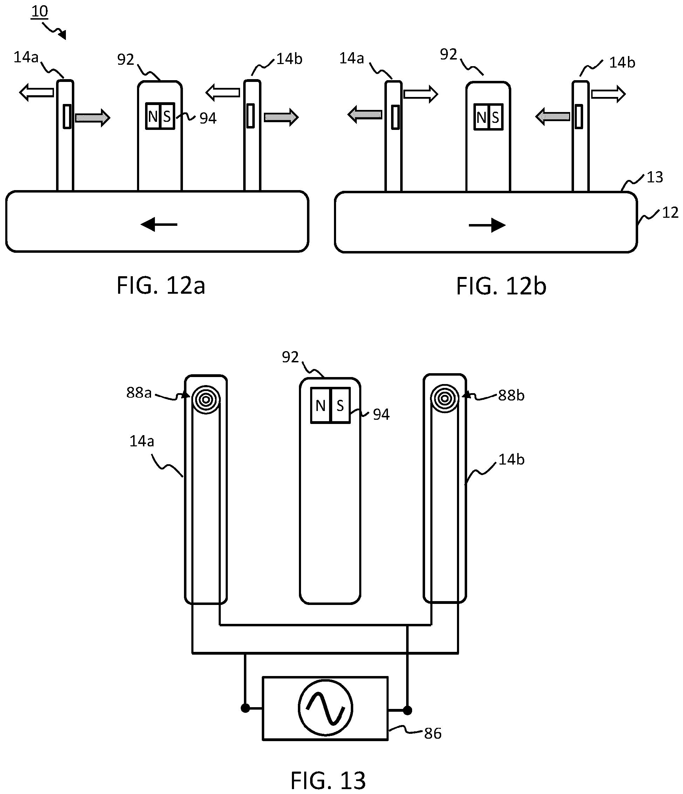

This is illustrated schematically in Fig. 12 which shows operation of the magnetic forcing arrangement in the high-intensity cleaning mode, where it is desired that the protruding members 14a, 14b are substantially stationary relative to the support surface 13. In the example of Fig. 12, the support body 12 is driven with a lateral oscillation, and the magnetic forcing arrangement is arranged to apply counterforces to counter lateral oscillation of the protruding members. However, the oscillation, and corresponding counter-forces, can be in other directions in further examples, for instance radial (rotational) oscillation and counterforces, or vertical or axial (up and down) oscillation and counterforces.

Fig. 12 illustrates the support surface being driven with a lateral oscillation. Fig. 12a shows the phase of the oscillation of the support body 12 in a first direction and Fig. 12b in a second opposite direction. This movement of the surface 13 (indicated by black arrows) in each phase exerts a corresponding lateral force on each of the protruding members (indicated by the white arrows). In the first phase (Fig. 12a), the loop, coil or planar solenoids 88a, 88b are driven with current in a first direction, thereby generating magnetic field induced counterforces (indicated by the shaded arrows) which pull the first protruding member 14a towards the central protruding member 92 and push the second protruding member 14b away from the central member 92. In the second phase (Fig. 12b), the loop, coil or planar solenoids 88a, 88b are driven with current in the opposite direction, thereby generating magnetic fields with forces (indicated by shaded arrows) which push the first protruding member 14a away from the central protruding member 92 and pull the second protruding member 14b towards from the central member 92. Thus the generated magnetic forces are controlled to counter the movement of the protruding members caused by the oscillating support surface. Preferably, the counter forces exactly match the oscillatory forces, so that the protruding members are left stationary (no oscillation).

In at least a second mode, the magnetic force mechanism may be deactivated. The second mode may be then a wide-area cleaning mode. Therefore, in the second mode, the protruding members 14a, 14b are free to oscillate due to the oscillation of the support surface 13 at their proximal ends 20, and thus the field intensity distribution is more spatially distributed or homogeneous.

In further embodiments, the magnetic forcing arrangement can be used by itself to induce a certain pattern of oscillatory motion of the protruding members 14a, 14b, without oscillation by the mechanical oscillation mechanism 82.

The solenoids 88a, 88b and an example driving circuitry of the solenoids is illustrated schematically in Fig. 13. For ease of illustration, the solenoid coils are shown facing out of the page. However, in practice the coils would each be arranged facing the central 92 protruding member containing the permanent magnet (i.e. at 90 degrees to orientation shown in Fig. 13).

Each solenoid 88a, 88b comprises a conductive coil which may be for example engraved or imprinted onto each protruding member 14a, 14b. The coil may be a flat planar coil, or may be tubular. It may be a single loop coil, or a multiple winding coil. Where the coil is engraved, an electrical insulating layer may be applied cover the coil to prevent interference with the RF electrodes 18. As shown, in this example, the first 88a and second 88b solenoids are electrically coupled together in parallel, and with the rails of the circuit connected to an oscillatory driver 86. Thus, driving of the solenoids is synchronous in this example (although asynchronous driving is also possible in alternative examples).

In advantageous examples, the driving of the magnetic forcing arrangement and the driving of the mechanical oscillation mechanism 82, e.g. drive train (which oscillates the support structure 12) are maintained in synchrony by controlling both using a shared oscillatory driver or signal generator. This oscillatory driver may be comprised by the mechanical oscillation mechanism itself, so that the magnetic forcing arrangement is driven by the driver of the oscillation mechanism. For example, in a powered toothbrush, the shared driver may be a drive train of the powered toothbrush.

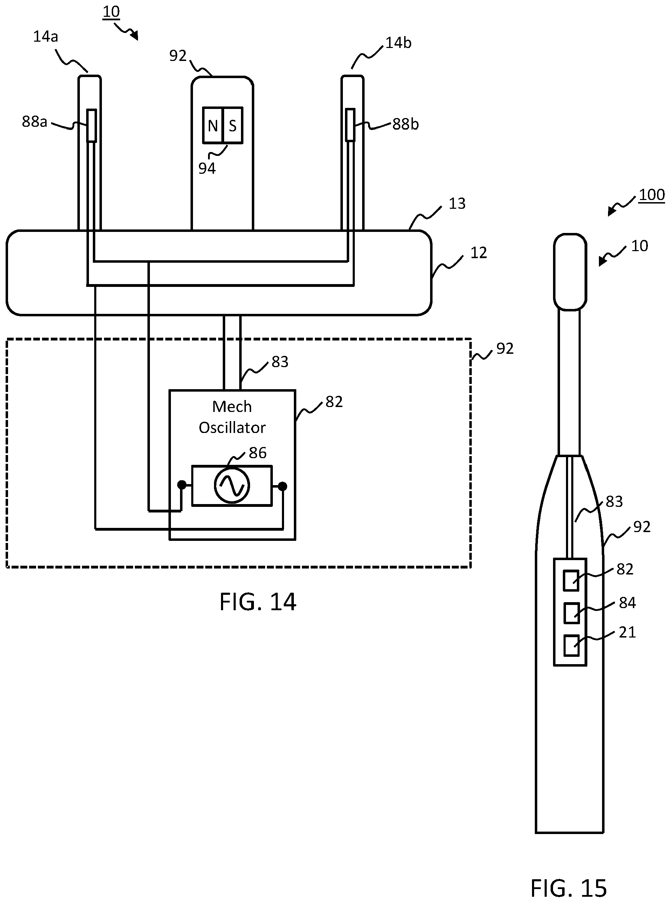

An example of this arrangement is schematically illustrated in Fig. 14.

As shown, the mechanical oscillator 82 comprises an electrical oscillatory signal generator 86 which generates a current which drives the motor of the mechanical oscillator to oscillate the support body. This signal generator is further coupled to the driving circuit of the magnetic forcing arrangement. Thus, the two are driven in synchrony with one another so that the magnetic forces generated by the solenoids 88a, 88b are always generated at the same time as the lateral oscillatory force, so that the one can counter or eliminate the other.

The magnetic forcing arrangement examples discussed above relate to examples of active magnetic forcing arrangements. However, passive options are also possible.

For example, the arrangement may comprise a conductive plate or sheet provided in place of each of the solenoids 88 in the example of Figs. 12-14 discussed above, with a planar surface arranged facing the permanent magnet. When such a conductive sheet is moved in a first direction relative to the permanent magnet, this induces eddy currents in the sheet, and these eddy currents induce a secondary magnetic field inducing a magnetic force counter to the direction of movement of the conductive sheet. Thus, this arrangement provides a passive eddy current damper which magnetically resists the movement of the protruding member caused by the oscillation of the support surface (by the mechanical oscillator 82).

Above have been described a number of embodiments in which the change between different modes of the device is achieved through mechanical means, comprising controlling a change in a position or movement pattern of the protruding members. This effects a change in the spatial intensity distribution of the resulting RF field.

According to one or more embodiments, any of the above mechanical approaches may be combined with any the electrical means for controlling the RF field intensity distribution. For example, different subsets of the protruding member electrodes may be selectively electrically activated or deactivated. For example, the controller may be arranged to selectively supply drive signals to electrodes of a subset of protruding members which are oscillating, while drive signals to a subset of electrodes that are controlled not to oscillate (e.g. through counterforces) are deactivated, or vice versa.

The oral cleaning and/or treatment device 100 may further comprise a control unit or body portion 92 mechanically and electrically coupled to the cleaning unit. The mechanical oscillation mechanism 82 may be housed in the body portion in this example. The RF generator 84 may also be housed in the body portion (not shown in Fig. 14). The controller 21 may further be provided in the body portion (not shown), operatively coupled to both the oscillation mechanism and the RF generator 84, and adapted to control configuration of the mode of the device.

It is noted that although in above-described examples, changing between the different modes is based on changing a movement pattern of the protruding members, this is not the only way of mechanically altering the RF field intensity distribution. Other examples could include simply a change in position of the protruding members, thereby altering a resultant field distribution, e.g. spreading the protruding members further apart to spread the RF field more widely, or pushing the protruding members closer together to achieve a more locally focused field. This could be achieved with suitable actuators for example. Suitable actuators may include for example piezoelectric actuator elements, ferroelectric polymer actuators, capacitive micromachined ultrasonic transducers (CMUT) actuator elements, electroactive polymer (EAP) actuation elements, electro-magnetorheological actuators, or any other suitable example.

As discussed, the oral cleaning and/or treatment device can take different forms. In one example, the device 100 is a toothbrush device. An example is schematically illustrated in Fig. 15. The cleaning unit 10 in this example is a brush head for the toothbrush.

The device further comprises a body portion 92 housing the RF generator 84 and, the body portion mechanically and electrically coupled to the cleaning unit 10. In the example depicted in Fig. 15, the body portion further houses the mechanical oscillation mechanism 82 where one is included. The body portion may further house the controller 21 operatively coupled to the RF generator and the mechanical oscillation mechanism, and adapted to control the switching between the different modes.

The body portion 92 is electrically and mechanically coupled to the cleaning unit 10 portion. Fig. 15 schematically shows the mechanical connector element 83 (e.g. drive shaft) which mechanically couples the oscillator mechanism 82 with the cleaning unit support body 12. The body portion 92 forms a handle for the device. An electrical connection is also provided between the head and the body portion.

Optionally, the cleaning unit 10 may be arranged to be releasably coupleable to the body portion 92, so that the cleaning unit is detachable from the body portion.

Other types of oral cleaning and/or treatment device may alternatively be provided, such as brushing mouthpiece devices which may not include a coupled handle or body portion.

By way of non-limiting example, example oral cleaning or treatment devices which may be provided in accordance with embodiments of the invention include:
- toothbrush devices (e.g. powered toothbrush);
- oral irrigators;
- flossing devices;
- combined brushing and flossing devices, e.g. a device adapted to simultaneously provide powered brushing and deliver a flow or jet of fluid; and
- mouthpiece devices, e.g. brushing mouthpiece devices.

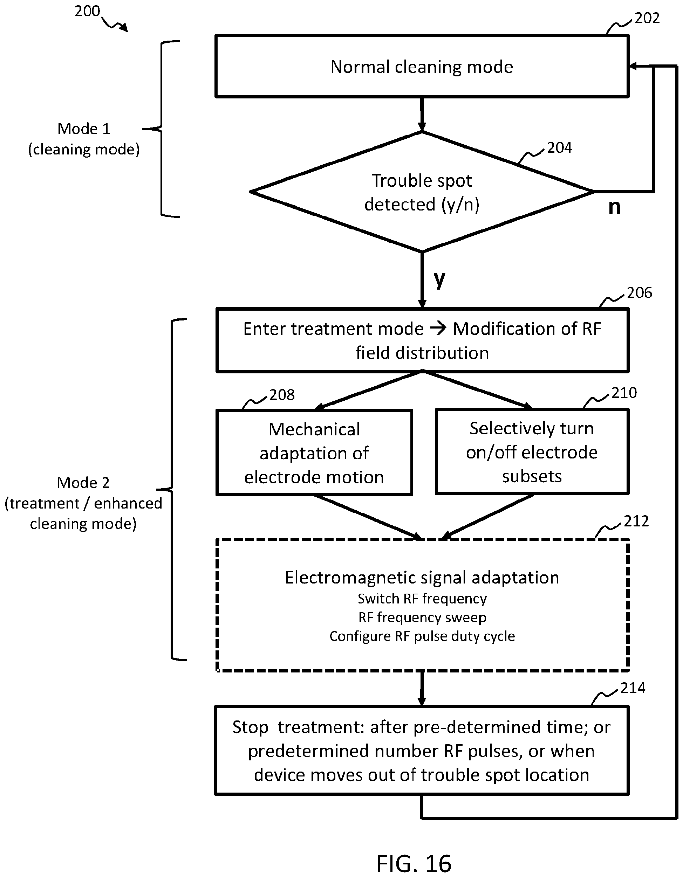

To further illustrate the principles of the present invention, Fig. 16 outlines an example workflow followed in accordance with an advantageous set of one or more embodiments. This workflow may for example be a control method implemented by the controller of the device according to one or more embodiments.

The cleaning or treatment device according to the present example is operable in at least a first mode ("Mode 1") which represents a standard cleaning mode, and at least a second mode ("Mode 2") which represents a treatment mode. The treatment mode may be for applying an adapted RF field (with adapted field shape) in a particular localized area of the mouth, for treating that area, e.g. more intense RF cleaning, or a different pattern or type of RF treatment.

The device starts by default in the first cleaning mode 202.

The controller is adapted to detect when the cleaning unit of the device is proximal a pre-determined region of interest, e.g. a trouble spot, known to require supplementary RF treatment. This may be based on use of a position and/or orientation sensor. Responsive to detecting that the cleaning unit is proximal the region of interest, the controller triggers 206 transition to the second mode ("Mode 2"). Alternatively, transition to the second mode may be triggered manually, based on a user input command from a user input element such as a button.

In the second mode, a spatial intensity distribution of the generated RF field is altered compared to the first mode. Modification of the RF field distribution can be achieved in different ways as discussed above. The change of field distribution can be achieved mechanically 208, based on altering a position or movement pattern of the protruding members. Additionally or alternatively, the change of field distribution can be achieved electrically, based on selectively activating or deactivating the electrodes of selected different subsets of the protruding members. The latter approach allows a particular spatial pattern of electrodes to be supplied with drive signals to generate RF field energy, such that a resultant RF field can be created with a desired resultant field distribution.

Following the mechanical 208 or electronic 210 adaptations to the field distribution or both, an optional further step 212 can be implemented comprising altering one or more further electrical characteristics of the RF field. This step can include one or more of:
implementing a cyclic sweep of RF frequencies;
changing a frequency of the RF field; and
implementing a pulsed RF field, and adjusting a duty cycle of the RF pulses.

The treatment mode ("Mode 2") with the altered RF field is continued until a pre-determined exit trigger is reached 214. For example, change back to the first mode may be triggered after a pre-determined time has elapsed, or until a certain quantity of RF energy has been delivered, e.g. a certain number of RF pulses have been delivered, or until the controller detects that the cleaning unit has moved away from the pre-determined region of interest (e.g. the trouble spot).

The controller can be implemented in numerous ways, with software and/or hardware, to perform the various functions required. A processor is one example of a controller which employs one or more microprocessors that may be programmed using software (e.g., microcode) to perform the required functions. The controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. An oral cleaning and treatment device (100) comprising:
a cleaning unit (10) comprising: a support body (12), two or more protruding members (14a, 14b), each extending outward from a support surface (13) of the support body, and each comprising one or more electrodes (18a, 18b), the protruding members being spaced from one another;
a radio-frequency (RF) generator (84) arranged to supply one or more drive signals to or between the electrodes of the two or more protruding members, in accordance with a drive scheme, to induce an RF alternating field in an area around and/or between the protruding members, to provide an oral cleaning function wherein the drive scheme includes at least one of the information of the drive signals to be supplied to individual electrodes, the pattern of drive signals to be supplied to the total set of electrodes, the pattern of electrodes which is active at any given time, and drive signal amplitudes of different electrodes at different points across the support body;
a controller (21) for selectively configuring the oral cleaning and/or treatment device in at least two different modes, a first mode in which the oral cleaning and/or treatment device is arranged for generating an RF field with a first spatial intensity distribution, and a second mode in which the oral cleaning and/or treatment device is arranged for generating an RF field with a second, different, spatial intensity distribution, wherein switching between the modes comprises controlling a change in a position or movement pattern of the protruding members using an actuator, and/or comprises controlling a change in characteristics of the drive signals or the drive scheme;
wherein each mode is arranged for generating an RF field with a different time-average spatial intensity distribution, over a defined time window; and
wherein in the first mode, the spatial intensity distribution is more spatially homogeneous than in a second mode.

2. An oral cleaning and treatment device as claimed in claim 1, wherein the controller (21) is adapted to switch between the modes based on:
controlling the actuator to control the change in a position or movement pattern of the protruding members; and/or
controlling the RF generator to implement the change in characteristics of the drive signal or the drive scheme.

3. An oral cleaning and treatment device as claimed in any of claims 1 or 2, further configured for generating an RF field intensity distribution which includes one or more intensity peaks or field gradients, and wherein in a first mode the peaks or field gradients are configured to move spatially relative to the support surface as a function of time, and in a second mode the peaks or field gradients are configured to exhibit reduced or no movement relative to the support surface.

4. An oral cleaning and treatment device as claimed in any of claims 1-3, wherein the RF generator is operable to independently control drive signals supplied to different subsets of the protruding members, and wherein changing between the modes comprises selectively activating or deactivating the electrodes of different subsets of the protruding members.

5. An oral cleaning and treatment device as claimed in any of claims 1-4, wherein
each of the protruding members is adapted to be flexible, to permit at least a distal end portion of the protruding member to flex or oscillate relative to a proximal end of the member,
the device further comprises an oscillation mechanism (82) arranged to induce oscillation of at least distal portions of the protruding members,
wherein switching between the at least two modes is based on adjusting a frequency of the oscillation of the protruding members.

6. An oral cleaning and treatment device as claimed in claim 5, wherein the oscillation mechanism (82) is arranged to oscillate the support surface (13) of the cleaning unit at an adjustable frequency to thereby induce the oscillation of the protruding members, and wherein switching between the modes comprises adjusting a frequency of oscillation of the support surface.

7. An oral cleaning and treatment device (100) as claimed in claim 6, wherein at least portions of each of the protruding members exhibit a natural frequency of oscillation relative to the proximal ends, and wherein, in a first mode, said frequency of oscillation of the support surface is set at a frequency which is resonant with said natural frequency, and in a second mode said frequency of oscillation of the support surface is set at a frequency which is not resonant with the natural frequency.

8. An oral cleaning and treatment device (100) as claimed in claim 7, wherein, in the second mode, the frequency of oscillation of the support surface is set at a frequency which is anti-resonant with the natural frequency of the protruding members.

9. An oral cleaning and treatment device (100) as claimed in any of claims 5-8,
wherein each of the protruding members has a proximal end connected to the support surface, and wherein a region of the support body meeting or surrounding the proximal end of each protruding member has a first set of mechanical resonance frequencies, and a remainder of the surface has a second set of mechanical resonance frequencies,
and wherein in a first mode, the support surface is driven to oscillate at a frequency resonant with a frequency of both the first and second frequency sets, and in a second mode is driven to oscillate at a frequency non-resonant with the first frequency set and resonant with a frequency of the second frequency range.

10. An oral cleaning and treatment device (100) as claimed in any of claims 1-9, further comprising a magnetic or electromagnetic forcing arrangement arranged for exerting a magnetic or electromagnetic force on each of the protruding members, and wherein the switching between the modes comprises adjusting a movement pattern of the protruding members based at least in part on use of the magnetic forcing arrangement.

11. An oral cleaning and treatment device (100) as claimed in claim 10, wherein the support surface is oscillated at a first frequency, and wherein, in one mode, the magnetic forcing arrangement is controlled to impart a pattern of counterforces on the protruding members in synchrony with the oscillation of the support surface, so as to counter or eliminate movement of the protruding members due to movement of the support surface.

12. An oral cleaning and treatment device as claimed in any of claims 1-11, wherein the controller (21) is adapted to determine a position or orientation of the cleaning unit, and wherein switching between the modes is triggered based on the determined position or orientation.

## Patentansprüche

1. Orale Reinigungs- und Behandlungsvorrichtung (100), umfassend:
eine Reinigungseinheit (10), umfassend: einen Trägerkörper (12), zwei oder mehr vorstehende Elemente (14a, 14b), die sich jeweils von einer Trägeroberfläche (13) des Trägerkörpers nach außen erstrecken und jeweils eine oder mehrere Elektroden (18a, 18b) umfassen, wobei die vorstehenden Elemente voneinander beabstandet sind;
einen Hochfrequenz- (HF) -Generator (84), der angeordnet ist, um in Übereinstimmung mit einem Ansteuerungsschema ein oder mehrere Ansteuerungssignale an oder zwischen die Elektroden der zwei oder mehr vorstehenden Elemente zu liefern, um ein HF-Wechselfeld in einem Bereich um und/oder zwischen den vorstehenden Elementen zu induzieren, um eine orale Reinigungsfunktion bereitzustellen, wobei das Ansteuerungsschema mindestens eine der Informationen der Ansteuerungssignale, die den individuellen Elektroden geliefert werden sollen, das Muster der Ansteuerungssignale, die dem gesamten Satz von Elektroden geliefert werden sollen, das Muster der Elektroden, das zu jeder gegebenen Zeit aktiv ist, und die Ansteuerungssignalamplituden verschiedener Elektroden an verschiedenen Punkten über den Trägerkörper einschließt;
eine Steuerung (21) zum selektiven Konfigurieren der oralen Reinigungs- und/oder Behandlungsvorrichtung in mindestens zwei verschiedenen Modi, einem ersten Modus, in dem die orale Reinigungs- und/oder Behandlungsvorrichtung zum Erzeugen eines HF-Feldes mit einer ersten räumlichen Intensitätsverteilung angeordnet ist, und einem zweiten Modus, in dem die orale Reinigungs- und/oder Behandlungsvorrichtung zum Erzeugen eines HF-Feldes mit einer zweiten, unterschiedlichen räumlichen Intensitätsverteilung angeordnet ist, wobei ein Umschalten zwischen den Modi ein Steuern einer Änderung einer Position oder eines Bewegungsmusters der vorstehenden Elemente unter Verwendung eines Aktuators umfasst, und/oder ein Steuern einer Änderung von Eigenschaften der Ansteuerungssignale oder des Ansteuerungsschemas umfasst;
wobei jeder Modus zum Erzeugen eines HF-Feldes mit einer unterschiedlichen zeitlich gemittelten räumlichen Intensitätsverteilung über ein definiertes Zeitfenster angeordnet ist; und
wobei im ersten Modus die räumliche Intensitätsverteilung räumlich homogener ist als in einem zweiten Modus.

2. Orale Reinigungs- und Behandlungsvorrichtung nach Anspruch 1, wobei die Steuereinheit (21) adaptiert ist, um zwischen den Modi basierend auf folgendem umzuschalten:
Steuern des Aktuators, um die Änderung einer Position oder eines Bewegungsmusters der vorstehenden Elemente zu steuern; und/oder
Steuern des HF-Generators, um die Änderung von Eigenschaften des Ansteuerungssignals oder des Ansteuerungsschemas zu implementieren.

3. Orale Reinigungs- und Behandlungsvorrichtung nach einem der Ansprüche 1 oder 2, weiter konfiguriert zum Erzeugen einer HF-Feldintensitätsverteilung, die eine oder mehrere Intensitätsspitzen oder Feldgradienten einschließt, und wobei in einem ersten Modus die Spitzen oder Feldgradienten konfiguriert sind, um sich als ein Funktion der Zeit räumlich relativ zur Trägeroberfläche zu bewegen, und in einem zweiten Modus die Spitzen oder Feldgradienten konfiguriert sind, um eine reduzierte oder keine Bewegung relativ zur Trägeroberfläche aufzuweisen.

4. Orale Reinigungs- und Behandlungsvorrichtung nach einem der Ansprüche 1-3, wobei der HF-Generator betrieben werden kann, um unabhängig Ansteuerungssignale zu steuern, die verschiedenen Untersätzen der vorstehenden Elemente geliefert werden, und wobei eine Änderung zwischen den Modi das selektive Aktivieren oder Deaktivieren der Elektroden verschiedener Untersätze der vorstehenden Elemente umfasst.

5. Orale Reinigungs- und Behandlungsvorrichtung nach einem der Ansprüche 1-4, wobei
jedes der vorstehenden Elemente adaptiert ist, um flexibel zu sein, um mindestens einem distalen Endabschnitt des vorstehenden Elements zu gestatten, sich relativ zu einem proximalen Ende des Elements zu biegen oder zu oszillieren,
die Vorrichtung weiter einen Oszillationsmechanismus (82) umfasst, der angeordnet ist, um eine Oszillation von mindestens distalen Abschnitten der vorstehenden Elemente zu induzieren,
wobei ein Umschalten zwischen den mindestens zwei Modi auf dem Anpassen einer Frequenz der Oszillation der vorstehenden Elemente basiert.

6. Orale Reinigungs- und Behandlungsvorrichtung nach Anspruch 5, wobei der Oszillationsmechanismus (82) angeordnet ist, um die Trägeroberfläche (13) der Reinigungseinheit mit einer anpassbaren Frequenz zu oszillieren, um dadurch die Oszillation der vorstehenden Elemente zu induzieren, und wobei ein Umschalten zwischen den Modi das Anpassen einer Frequenz der Oszillation der Trägeroberfläche umfasst.

7. Orale Reinigungs- und Behandlungsvorrichtung (100) nach Anspruch 6, wobei mindestens Abschnitte jedes der vorstehenden Elemente eine Eigenfrequenz der Oszillation relativ zu den proximalen Enden aufweisen, und wobei, in einem ersten Modus, die Frequenz der Oszillation der Trägeroberfläche auf eine Frequenz eingestellt ist, die mit der Eigenfrequenz in Resonanz ist, und in einem zweiten Modus die Frequenz der Oszillation der Trägeroberfläche auf eine Frequenz eingestellt ist, die nicht mit der Eigenfrequenz in Resonanz ist.

8. Orale Reinigungs- und Behandlungsvorrichtung (100) nach Anspruch 7, wobei in dem zweiten Modus die Frequenz der Oszillation der Trägeroberfläche auf eine Frequenz eingestellt ist, die in Anti-Resonanz mit der Eigenfrequenz der vorstehenden Elemente ist.

9. Orale Reinigungs- und Behandlungsvorrichtung (100) nach einem der Ansprüche 5-8,
wobei jedes der vorstehenden Elemente ein proximales Ende aufweist, das mit der Trägeroberfläche verbunden ist, und wobei ein Gebiet des Trägerkörpers, das auf das proximale Ende jedes vorstehenden Elements trifft oder dieses umgibt, einen ersten Satz mechanischer Resonanzfrequenzen aufweist, und ein Rest der Oberfläche einen zweiten Satz mechanischer Resonanzfrequenzen aufweist,
und wobei in einem ersten Modus die Trägeroberfläche angesteuert wird, um mit einer Frequenz zu oszillieren, die mit einer Frequenz sowohl des ersten als auch des zweiten Frequenzsatzes in Resonanz ist, und in einem zweiten Modus angesteuert wird, um mit einer Frequenz zu oszillieren, die mit dem ersten Frequenzsatz nicht in Resonanz ist und mit einer Frequenz des zweiten Frequenzbereichs in Resonanz ist.

10. Orale Reinigungs- und Behandlungsvorrichtung (100) nach einem der Ansprüche 1-9, weiter umfassend eine magnetische oder elektromagnetische Zwangsanordnung, die zum Ausüben einer magnetischen oder elektromagnetischen Kraft auf jedes der vorstehenden Elemente angeordnet ist, und wobei das Umschalten zwischen den Modi ein Anpassen eines Bewegungsmusters der vorstehenden Elemente basierend zumindest teilweise auf der Verwendung der magnetischen Zwangsanordnung umfasst.

11. Orale Reinigungs- und Behandlungsvorrichtung (100) nach Anspruch 10, wobei die Trägeroberfläche mit einer ersten Frequenz oszilliert, und wobei, in einem Modus, die magnetische Zwangsanordnung gesteuert wird, um den vorstehenden Elementen synchron mit der Oszillation der Trägeroberfläche ein Muster von Gegenkräften zu vermitteln, um der Bewegung der vorstehenden Elemente aufgrund der Bewegung der Trägeroberfläche entgegenzuwirken oder sie zu eliminieren.

12. Orale Reinigungs- und Behandlungsvorrichtung nach einem der Ansprüche 1-11, wobei die Steuereinheit (21) adaptiert ist, um eine Position oder Orientierung der Reinigungseinheit zu bestimmen, und wobei ein Umschalten zwischen den Modi basierend auf der bestimmten Position oder Orientierung ausgelöst wird.

## Revendications

1. Dispositif de nettoyage et de traitement buccal (100) comprenant :
une unité de nettoyage (10) comprenant : un corps de support (12), deux, ou plus, éléments saillants (14a, 14b), chacun s'étendant vers l'extérieur à partir d'une surface de support (13) du corps de support, et comprenant chacun une ou plusieurs électrodes (18a, 18b), les éléments saillants étant espacés les uns des autres ;
un générateur de radiofréquences (RF) (84) agencé pour fournir un ou plusieurs signaux d'entraînement vers, ou entre, les électrodes des deux, ou plus, éléments saillants, selon un schéma d'entraînement, pour induire un champ alternatif RF dans une zone autour, et/ou entre, les éléments saillants, pour fournir une fonction de nettoyage buccal, dans lequel le schéma d'entraînement inclut au moins des informations des signaux d'entraînement à fournir aux électrodes individuelles, du motif de signaux d'entraînement à fournir à l'ensemble total d'électrodes, du motif d'électrodes qui sont actives à un moment donné, et d'amplitudes de signaux d'entraînement de différentes électrodes à différents points à travers le corps de support ;
un dispositif de commande (21) pour configurer de manière sélective le dispositif de nettoyage et/ou de traitement buccal dans au moins deux modes différents, un premier mode dans lequel le dispositif de nettoyage et/ou de traitement buccal est agencé pour générer un champ RF avec une première distribution d'intensité spatiale, et un second mode dans lequel le dispositif de nettoyage et/ou de traitement buccal est agencé pour générer un champ RF avec une seconde distribution d'intensité spatiale différente, dans lequel la commutation entre les modes comprend la commande d'un changement d'un motif de position ou de mouvement des éléments saillants à l'aide d'un actionneur, et/ou comprend la commande d'un changement de caractéristiques des signaux d'entraînement ou du schéma d'entraînement ;
dans lequel chaque mode est agencé pour générer un champ RF avec une différente distribution d'intensité spatiale moyenne dans le temps, sur une fenêtre temporelle définie ; et
dans lequel, dans le premier mode, la distribution d'intensité spatiale est plus homogène spatialement que dans un second mode.

2. Dispositif de nettoyage et de traitement buccal selon la revendication 1, dans lequel le dispositif de commande (21) est adapté pour commuter entre les modes sur la base de :
la commande de l'actionneur pour commander le changement d'un motif de position ou de mouvement des éléments saillants ; et/ou
la commande du générateur RF pour mettre en oeuvre le changement de caractéristiques du signal d'entraînement ou du schéma d'entraînement.

3. Dispositif de nettoyage et de traitement buccal selon l'une quelconque des revendications 1 ou 2, configuré en outre pour générer une distribution d'intensité de champ RF qui inclut un ou plusieurs pics d'intensité ou gradients de champ, et dans lequel, dans un premier mode, les pics ou les gradients de champ sont configurés pour se déplacer spatialement par rapport à la surface de support en fonction du temps et, dans un second mode, les pics ou les gradients de champ sont configurés pour présenter un mouvement réduit ou aucun mouvement par rapport à la surface de support.

4. Dispositif de nettoyage et de traitement buccal selon l'une quelconque des revendications 1-3, dans lequel le générateur RF peut fonctionner pour commander indépendamment des signaux d'entraînement fournis à différents sous-ensembles des éléments saillants et dans lequel le changement entre les modes comprend l'activation ou la désactivation sélective des électrodes de différents sous-ensembles des éléments saillants.

5. Dispositif de nettoyage et de traitement buccal selon l'une quelconque des revendications 1-4, dans lequel
chacun des éléments saillants est adapté pour être flexible, pour permettre à au moins une partie d'extrémité distale de l'élément saillant de fléchir ou d'osciller par rapport à une extrémité proximale de l'élément,
le dispositif comprend en outre un mécanisme d'oscillation (82) agencé pour induire une oscillation d'au moins des parties distales des éléments saillants,
dans lequel la commutation entre les au moins deux modes est basée sur l'ajustement d'une fréquence d'oscillation des éléments saillants.

6. Dispositif de nettoyage et de traitement buccal selon la revendication 5, dans lequel le mécanisme d'oscillation (82) est agencé pour faire osciller la surface de support (13) de l'unité de nettoyage à une fréquence ajustable pour induire, de ce fait, l'oscillation des éléments saillants, et dans lequel la commutation entre les modes comprend l'ajustement d'une fréquence d'oscillation de la surface de support.

7. Dispositif de nettoyage et de traitement buccal (100) selon la revendication 6, dans lequel au moins des parties de chacun des éléments saillants présentent une fréquence propre d'oscillation par rapport aux extrémités proximales et dans lequel, dans un premier mode, ladite fréquence d'oscillation de la surface de support est réglée à une fréquence qui est en résonance avec ladite fréquence propre et, dans un second mode, ladite fréquence d'oscillation de la surface de support est réglée à une fréquence qui n'est pas en résonance avec la fréquence propre.

8. Dispositif de nettoyage et de traitement buccal (100) selon la revendication 7, dans lequel, dans le second mode, la fréquence d'oscillation de la surface de support est réglée à une fréquence qui est anti-résonnante avec la fréquence propre des éléments saillants.

9. Dispositif de nettoyage et de traitement buccal (100) selon l'une quelconque des revendications 5-8,
dans lequel chacun des éléments saillants présente une extrémité proximale reliée à la surface de support et dans lequel une région du corps de support rencontrant ou entourant l'extrémité proximale de chaque élément saillant présente un premier ensemble de fréquences de résonance mécanique, et un reste de la surface présente un second ensemble de fréquences de résonance mécanique,
et dans lequel, dans un premier mode, la surface de support est entraînée pour osciller à une fréquence de résonance avec une fréquence à la fois du premier et du second ensembles de fréquences et, dans un second mode, elle est entraînée pour osciller à une fréquence non résonnante avec le premier ensemble de fréquences et résonnante avec une fréquence de la seconde plage de fréquences.

10. Dispositif de nettoyage et de traitement buccal (100) selon l'une quelconque des revendications 1-9, comprenant en outre un agencement de forçage magnétique ou électromagnétique agencé pour exercer une force magnétique ou électromagnétique sur chacun des éléments saillants et dans lequel la commutation entre les modes comprend l'ajustement d'un motif de mouvement des éléments saillants sur la base, au moins en partie, de l'utilisation de l'agencement de forçage magnétique.

11. Dispositif de nettoyage et de traitement buccal (100) selon la revendication 10, dans lequel la surface de support oscille à une première fréquence et dans lequel, dans un mode, l'agencement de forçage magnétique est commandé pour conférer un motif de forces contraires aux éléments saillants en synchronisation avec l'oscillation de la surface de support, de manière à contrecarrer ou éliminer le mouvement des éléments saillants dû au mouvement de la surface de support.

12. Dispositif de nettoyage et de traitement buccal selon l'une quelconque des revendications 1-11, dans lequel le dispositif de commande (21) est adapté pour déterminer une position ou une orientation de l'unité de nettoyage et dans lequel la commutation entre les modes est déclenchée sur la base de la position ou de l'orientation déterminée.
